# FASCICULE DE BREVET EUROPEEN

(11) **EP 4 153 203 B1**
(45) Date de publication et mention de la délivrance du brevet: **18.12.2024**
(21) Numéro de dépôt: 21732475.5
(22) Date de dépôt: 18.05.2021
(51) Int. Cl.: A61K 36/064, A61K 36/9066, A61K 31/05, A61P 15/00

(54) **COMPOSITIONS ET COMBINAISONS DESTINÉES AUX SUJETS SOUFFRANT D'ENDOMÉTRIOSE**
ZUSAMMENSETZUNGEN UND KOMBINATIONEN FÜR PERSONEN MIT ENDOMETRIOSE
COMPOSITIONS AND COMBINATIONS FOR SUBJECTS SUFFERING FROM ENDOMETRIOSIS

(30) Priorité: 19.05.2020 FR 2005097
(43) Date de publication de la demande: 29.03.2023
(73) Titulaire: Gynov, 33000 Bordeaux (FR)
(72) Inventeur: MOUSSET, Pierre-Yves, 33360 LATRESNE (FR)
(74) Mandataire: Cabinet Becker et Associés
(86) Numéro de dépôt international: PCT/FR2021/050864
(87) Numéro de publication internationale: WO 2021/234271

(56) Documents cités:
- EP-A1- 2 617 425
- EP-A1- 3 338 769
- WO-A1-99/01148
- US-A1- 2012 251 500
- US-A1- 2019 192 588
- ARABLOU TAHEREH ET AL: "Curcumin and endometriosis: Review on potential roles and molecular mechanisms", BIOMEDICINE AND PHARMACOTHERAPY, ELSEVIER, FR, vol. 97, 6 November 2017 (2017-11-06), pages 91 - 97, XP085323912, ISSN: 0753-3322, DOI: 10.1016/J.BIOPHA.2017.10.119
- JANA SAYANTAN ET AL: "Curcumin as anti-endometriotic agent: Implication of MMP-3 and intrinsic apoptotic pathway", BIOCHEMICAL PHARMACOLOGY, vol. 83, no. 6, 2012, pages 797 - 804, XP028890384, ISSN: 0006-2952, DOI: 10.1016/J.BCP.2011.12.030
- BOROUMAND SAFIEH ET AL: "Curcumin-loaded nanofibers for targeting endometriosis in the peritoneum of a mouse model", JOURNAL OF MATERIALS SCIENCE: MATERIALS IN MEDICINE, SPRINGER NEW YORK LLC, UNITED STATES, vol. 31, no. 1, 14 December 2019 (2019-12-14), XP036994040, ISSN: 0957-4530, [retrieved on 20191214], DOI: 10.1007/S10856-019-6337-4
- HARRIS T VLASS AM: "Can Herbal Medicines Improve Cellular Immunity Patterns in Endometriosis?", MEDICINAL AND AROMATIC PLANTS, vol. 04, no. 02, 1 January 2014 (2014-01-01), XP055415962, DOI: 10.4172/2167-0412.1000184
- KOLAHDOUZ MOHAMMADI ROYA ET AL: "Resveratrol and endometriosis: In vitro and animal studies and underlying mechanisms (Review)", BIOMEDICINE AND PHARMACOTHERAPY, ELSEVIER, FR, vol. 91, 28 April 2017 (2017-04-28), pages 220 - 228, XP085060089, ISSN: 0753-3322, DOI: 10.1016/J.BIOPHA.2017.04.078
- DATABASE MEDLINE [online] US NATIONAL LIBRARY OF MEDICINE (NLM), BETHESDA, MD, US; 13 February 2019 (2019-02-13), DULL ANA-MARIA ET AL: "Therapeutic Approaches of Resveratrol on Endometriosis via Anti-Inflammatory and Anti-Angiogenic Pathways.", XP002801326, Database accession no. NLM30781885
- MOLECULES (BASEL, SWITZERLAND) 13 FEB 2019, vol. 24, no. 4, 13 February 2019 (2019-02-13), ISSN: 1420-3049
- THER ADV: "Therapeutic Advances in Gastroenterology Review", 1 January 2012 (2012-01-01), pages 111 - 125, XP055755857, Retrieved from the Internet <URL:https://www.ncbi.nlm.nih.gov/pmc/articles/PMC3296087/pdf/10.1177_1756283X11428502.pdf>
- LASCHKE MATTHIAS W ET AL: "The gut microbiota: a puppet master in the pathogenesis of endometriosis?", AMERICAN JOURNAL OF OBSTETRICS & GYNECOLOGY, MOSBY, ST LOUIS, MO, US, vol. 215, no. 1, 18 February 2016 (2016-02-18), XP029619236, ISSN: 0002-9378, DOI: 10.1016/J.AJOG.2016.02.036
- BAKER JAMES M ET AL: "Estrogen-gut microbiome axis: Physiological and clinical implications", MATURITAS, vol. 103, 2017, pages 45 - 53, XP085149460, ISSN: 0378-5122, DOI: 10.1016/J.MATURITAS.2017.06.025

## Description

### DOMAINE TECHNIQUE

La présente invention se rapporte au domaine de la santé et concerne plus particulièrement le traitement et la prise en charge de l'endométriose ainsi que des symptômes et des troubles associés à l'endométriose.

### ARRIERE PLAN TECHNOLOGIQUE

L'endométriose se définit par la présence de tissu endométrial en dehors de la cavité utérine. Ce tissu peut se retrouver notamment au niveau des parois de l'utérus, des trompes de Fallope, des ovaires, des ligaments utérosacrés mais aussi au niveau de la vessie et du côlon. Plusieurs organes peuvent être ainsi touchés chez une même patiente. Dans de rares cas, des lésions d'endométriose peuvent également être présentes au niveau d'organes localisés à distance de l'utérus, par exemple dans les poumons. Ce tissu extra-utérin possède les mêmes caractéristiques que celui de l'endomètre et se comporte de la même façon sous l'influence des hormones ovariennes. Dans ce cas, les saignements menstruels n'ont aucune issue vers l'extérieur du corps. Les problèmes causés par ce sang piégé, qui entraine des inflammations des tissus voisins, sont divers : lésions, kystes, adhérence entre les organes.

Chez un sujet souffrant d'endométriose, on peut ainsi observer des lésions nodulaires superficielles à la surface des ovaires ou du péritoine, des kystes ovariens (endométriomes) mais aussi des nodules profonds qui envahissent des zones telles que la zone recto-vaginale, la vessie et les ligaments utérosacrés et des adhérences affectant le pelvis pouvant conduire à un pelvis gelé.

L'endométriose est polymorphique : les atteintes organiques et les symptômes et troubles associés sont très variables d'un sujet à l'autre. Il existe une classification permettant de classer l'endométriose en 4 stades en fonction du nombre de lésions et des organes atteints. Néanmoins, il n'existe pas une corrélation claire entre le stade de l'endométriose (I-IV) et son impact sur la fertilité et les douleurs associées.

En effet, les principaux symptômes et troubles associés à l'endométriose sont la douleur et l'infertilité. Le symptôme majeur est une douleur pelvienne récurrente parfois très aiguë, notamment au moment des règles (dysménorrhée). Ce caractère cyclique est évocateur de la maladie. Les lésions sont en effet sensibles aux hormones féminines et se comportent comme du tissu utérin. Les lésions vont donc proliférer, saigner et laisser des cicatrices fibreuses à chaque cycle menstruel. Chez certaines patientes, une importante innervation des lésions pourrait contribuer aux douleurs extrêmes parfois ressenties. En dehors de la période des règles, les patientes peuvent également souffrir au moment de l'ovulation, lors des rapports sexuels (dyspareunie) ou encore lorsqu'elles urinent ou défèquent.

Le second symptôme sont les troubles de la fertilité. En effet, une partie importante des patientes souffrant d'endométriose présente une hypofertilité ou une infertilité.

L'endométriose est de plus une pathologie fréquente, ayant un impact socioéconomique très important. Cette pathologie touche au minimum entre 10 et 15% des femmes. En France, selon une étude de l'Insee (2014), entre 2,1 et 4,2 millions de femmes seraient touchées par l'endométriose contre 3 millions pour les cancers et 2,7 millions pour le diabète de type 2.

L'endométriose peut affecter de manière très significative la vie des patientes, en particulier en raison des douleurs très intenses qu'elle provoque qui peuvent conduire à des arrêts de travail et à de l'absentéisme et en raison de l'infertilité et de des dyspareunies qui troublent la vie intime et familiale. On estime à environ 9,5 milliards d'euros par an le coût de l'endométriose en France, ce chiffre incluant à la fois les coûts directs (les soins des patientes) mais également les coûts indirects ayant un impact sur la productivité (les arrêts maladies). Le nombre d'hospitalisation en lien avec l'endométriose a également progressé de + 7,5% entre 2010 et 2013 (Petit, 2016).

L'étiologie de l'endométriose reste à l'heure actuelle inconnue et il n'existe pas de traitement curatif de la maladie.

Le traitement de l'endométriose réside dans la prise en charge de la douleur et de l'infertilité ainsi que la stabilisation des lésions. En plus des traitements antidouleurs (essentiellement basés sur la prise ponctuelle d'anti-inflammatoires non stéroïdiens (AINS)), le traitement de première intention est généralement un traitement hormonal destiné à supprimer les menstruations et basé sur la prise en continu d'un contraceptif oral progestatif (type désogestrel) ou oestroprogestatif ou sur l'utilisation d'un implant ou stérilet progestatif. Ce type de traitement peut permettre de réduire les douleurs liées à la réponse hormonale des lésions d'endométriose et peut permettre de stabiliser les lésions, voire de diminuer légèrement leur volume. Toutefois, il ne permet pas leur élimination totale. D'autres traitements peuvent être utilisés pour instaurer une ménopause artificielle, mais ces traitements ne sont généralement prescrits que pour une durée courte en raison de leurs effets secondaires. Ce type de traitements englobe les analogues de GnRH (leuprolide, triptoréline) et les anti-androgènes tels que le danazol qui sont généralement utilisés sur une période d'au plus 3 à 6 mois, par exemple en pré ou post-opératoire.

En France, la prise en charge de l'infertilité, en première intention, repose essentiellement sur les techniques de reproduction médicament assistées, notamment sur la fécondation in vitro.

En cas d'échec dans la prise en charge médicamenteuse de la douleur ou de l'infertilité, ou dans le cas où les nodules deviennent trop invasifs, le traitement de seconde intention est la chirurgie. Néanmoins, l'exérèse des lésions associées à l'endométriose peut se révéler délicate et très invasive, notamment dans des zones telles que le rectosigmoïde. Les récidives suite à une chirurgie sont également fréquentes, notamment en raison de l'impossibilité de supprimer toutes ou totalement les lésions présentes.

Il existe donc, à l'heure actuelle, un besoin pour de nouvelles stratégies de prise en charge des patientes souffrant d'endométriose.

### RESUME DE L'INVENTION

L'invention est telle que définie dans les revendications.

L'invention a pour objet une combinaison d'une levure de l'espèce *Saccharomyces cerevisiae* de variété boulardii de resvératrol et d'un extrait de curcuma comprenant au moins 15% en poids de curcuminoïdes, pour une utilisation dans le traitement ou la prévention des lésions d'endométriose chez un sujet souffrant ou susceptible de souffrir d'endométriose, par exemple pour ralentir le développement ou pour diminuer les lésions d'endométriose. La combinaison selon l'invention peut être en outre utilisée dans le traitement ou la prévention d'un ou plusieurs symptômes ou troubles associés à l'endométriose choisis dans le groupe constitué par l'hypofertilité, l'infertilité, les douleurs pelviennes notamment les dysménorrhées et dyspareunies, les douleurs cataméniales notamment au niveau urinaire ou intestinale, les troubles urinaires et intestinaux, les métrorrhagies, les rectorragies, l'inflammation chronique péritonéale, l'hyperperméabilité intestinale, la fatigue chronique et les combinaisons de ceux-ci. La combinaison selon l'invention peut de plus être utilisée pour normaliser la perméabilité intestinale, moduler le statut immunitaire, maintenir la fertilité et/ou diminuer le stress oxydatif chez le sujet souffrant ou susceptible de souffrir d'endométriose.

La levure de l'espèce *Saccharomyces cerevisiae* de variété boulardii, le resvératrol et l'extrait de curcuma comprenant au moins 15% en poids de curcuminoïdes peuvent être contenus dans une seule composition pharmaceutique, nutraceutique ou alimentaire ou répartis dans plusieurs compositions pharmaceutiques, nutraceutiques ou alimentaires séparées destinées à être administrées simultanément ou séquentiellement.

Dans certains modes de réalisations, le sujet souffre d'endométriose et a subi ou va subir un traitement chirurgical d'exérèse des lésions d'endométriose, un protocole médicamenteux de procréation médicalement assisté, notamment de fécondation in vitro et/ou est sous traitement contraceptif.

Dans d'autres modes de réalisation, la combinaison selon l'invention est incluse dans un complément alimentaire, éventuellement avec une ou plusieurs substances à but physiologique ou nutritionnel choisis de préférence parmi les vitamines, les oligoéléments, les minéraux, les prébiotiques, les probiotiques, les antioxydants, les acides aminés et leurs combinaisons, et de manière plus préférée parmi l'acide folique et l'acide 5-methyltetrahydrofolique, la vitamine B6, la vitamine B12, le zinc, le magnésium, le calcium, le sélénium, le manganèse, l'inuline, les fructo/galacto-oligosaccharides, la gomme d'acacia, la gomme guar, l'inositol et leurs combinaisons.

Selon un aspect supplémentaire, l'invention a pour objet une composition pharmaceutique, nutraceutique ou alimentaire, de préférence sous la forme d'un complément alimentaire, comprenant une combinaison de levures *Saccharomyces cerevisiae,* de resvératrol et un extrait de curcuma comprenant au moins 15% en poids de curcuminoïdes, selon les rapports suivants:
- de 1 à 50% en poids de levure *Saccharomyces cerevisiae* de variété boulardii,
- de 10 à 50 % en poids de resvératrol,
- de 1 à 50% par exemple de 1% à 20% ou de 10 à 50% en poids d'extrait de curcuma comprenant au moins 15% en poids de curcuminoïdes,
pour 100% en poids de la somme totale de resvératrol, de curcumine et de Saccharomyces *cerevisiae* de variété boulardii ou pour 100% en poids total de la composition, ladite composition étant de préférence adaptée pour une utilisation dans la prise en charge des sujets souffrant ou susceptibles de souffrir d'endométriose.

Cette composition peut, de plus, comprendre un ou plusieurs prébiotiques ; et/ou des vitamines, minéraux et/ou oligo-élements ; et/ou des poudres ou extraits de plantes, fruits, légumes, algues ou champignons ; et/ou un ou plusieurs probiotiques, de préférence ledit probiotique n'étant pas une bactérie du genre Lactobacillus.

La composition selon l'invention peut se présenter sous la forme d'unités de dose, par exemple sous la forme de comprimés ou de gélules, chaque unité de dose comprenant :
- de 10 à 300 mg, de préférence de 25 à 250 mg de resvératrol,
- de 1 mg à 300 mg voire de 10 à 300 mg, de préférence de 5 mg à 25 mg d'un extrait de curcuma comprenant au moins 15% en poids de curcuminoïdes ou de 25 à 250 mg d'un extrait de curcuma comprenant au moins 15% en poids de curcuminoïdes, et
- de 1×10⁷ CFU à 1×10¹¹ CFU, de préférence 1×10⁸ CFU à 1×10⁹ CFU de levure *Saccharomyces cerevisiae* de variété boulardii

La composition peut en outre comprendre de l'inuline et du zinc. Par exemple, la composition selon l'invention peut être caractérisée en ce qu'elle se présente sous la forme d'unités de dose comprenant :
- de 30 mg à 70 mg de resvératrol, de préférence trans-resvératrol,
- de 5 à 25 mg de curcuminoïdes sous la forme d'un extrait de curcuma associé à une cyclodextrine,
- de 1×10⁸ CFU à 1×10¹⁰ CFU, de préférence de 2×10⁸ CFU à 6×10⁸ CFU,par exemple 3×10⁸ CFU à 5×10⁸ CFU de levure *Saccharomyces cerevisiae* de variété boulardii,
- en option de 10 mg à 100 mg, de préférence de 25 mg à 50 mg d'inuline, et
- en option de 1 mg à 15 mg de zinc (Zn2+) de préférence de 2 mg à 8 mg de zinc, par exemple introduit sous forme de bisglycinate de zinc.

L'invention a également pour objet un kit pharmaceutique, nutraceutique ou alimentaire comprenant l'association de deux compositions :
- une première composition comprenant une levure *Saccharomyces cerevisiae* de variété boulardii de préférence en une quantité de 1×10⁷ CFU à 1×10¹¹ CFU et
- une seconde composition comprenant du resvératrol de préférence en une quantité de 10 à 300 mg et un extrait de curcuma en une quantité de 1 à 300 mg, e.g. de 10 à 300 mg, ledit extrait de curcuma comprenant au moins 15% en poids de curcuminoïdes ledit kit étant pour une utilisation chez des sujets souffrant ou susceptibles de souffrir d'endométriose.

La combinaison, composition ou kit selon l'invention peuvent être en plus caractérisé(e) en ce que :
- l'extrait de resvératrol est apporté sous la forme d'un extrait végétal enrichi en trans-resvératrol, par exemple un extrait de renouée du Japon titré à au moins 95% en poids de trans-resvératrol et/ou
- l'extrait de curcuma comprend de la curcumine, de la déméthoxycurcumine et de la bisdéméthoxycurcumine, et/ou
- l'extrait de curcuma comprend au moins 55%, de préférence au moins 60% en poids de curcumine par rapport au poids total des curcuminoïdes présents dans l'extrait, et/ou
- l'extrait de curcuma est encapsulé dans une cyclodextrine, de préférence une gamma cyclodextrine, et/ou

### DESCRIPTION DES FIGURES

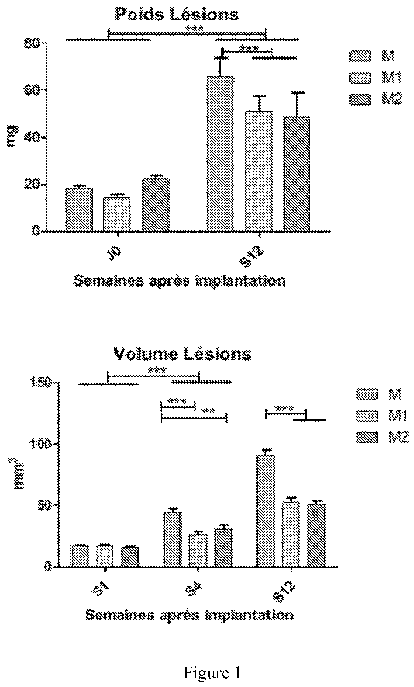

La Figure 1 montre la croissance, le poids et les volumes des lésions d'endométriose murine au cours du temps. M (contrôle) : groupe traité avec un mélange de resvératrol et d'extrait de curcuma ; M1 (invention) : groupe traité avec un mélange de resvératrol, d'extrait de curcuma et *S. boulardii* ; M2 (invention) : groupe traité avec un mélange de resvératrol, d'extrait de curcuma, *S. boulardii* et *L. acidophilus.*

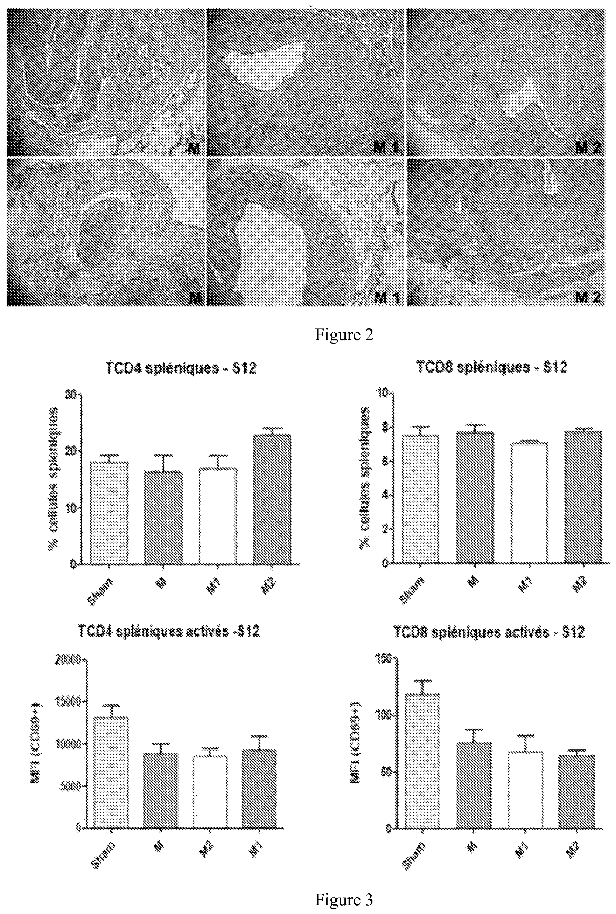

La Figure 2 montre l'histologie des lésions prélevées au sacrifice (coloration HE) pour les différents groupes M, M1 et M2.
La Figure 3 montre le statut immunitaire splénique des différents groupes après 12 semaines de traitement (S 12) - Lymphocytes T. SHAM (contrôle) correspond aux animaux pseudo-opérés ; M (contrôle) : groupe traité avec un mélange de resvératrol et d'extrait de curcuma ; M1 (invention) : groupe traité avec un mélange de resvératrol, d'extrait de curcuma et *S*. *boulardii* ; M2 (invention) : groupe traité avec un mélange de resvératrol, d'extrait de curcuma, *S. boulardii* et *L. acidophilus.*

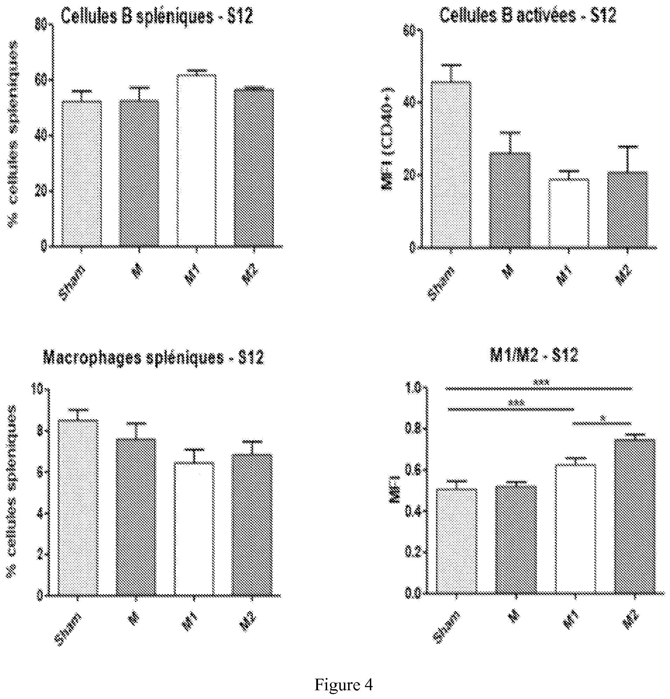

La Figure 4 montre le statut immunitaire splénique des différents groupes après 12 semaines de traitement (S 12) - Lymphocytes B et Macrophages. SHAM correspond aux animaux pseudo-opérés ;
M (contrôle) : groupe traité avec un mélange de resvératrol et d'extrait de curcuma ; M1 (invention) : groupe traité avec un mélange de resvératrol, d'extrait de curcuma et *S*. *boulardii* ; M2 (invention) : groupe traité avec un mélange de resvératrol, d'extrait de curcuma, *S. boulardii* et *L. acidophilus.*

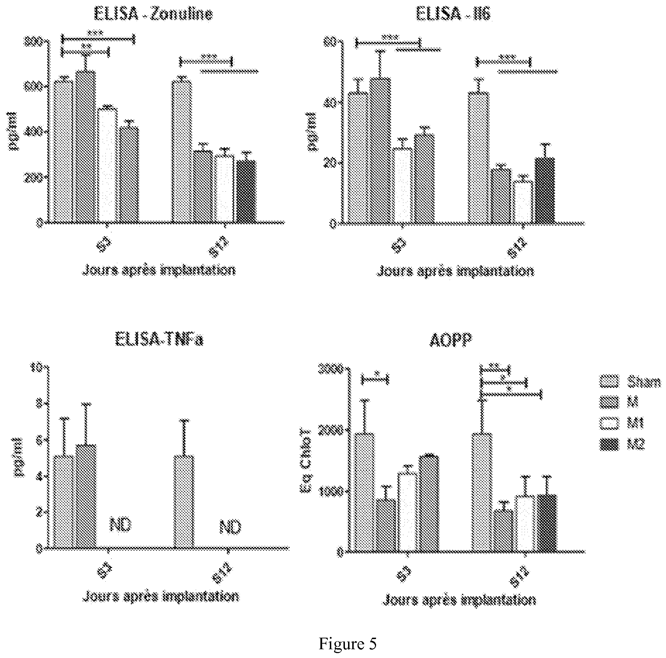

La Figure 5 montre l'effet sur la zonuline, l'interleukine 6 (IL6), le TNFα et les produits avancés protéiques d'oxydation (AOPP). SHAM (contrôle) correspond aux animaux pseudo-opérés ; M (contrôle) : groupe traité avec un mélange de resvératrol et d'extrait de curcuma ; M1 (invention) : groupe traité avec un mélange de resvératrol, d'extrait de curcuma et *S*. *boulardii* ; M2 (invention) : groupe traité avec un mélange de resvératrol, d'extrait de curcuma, *S. boulardii* et *L. acidophilus.*

### DESCRIPTION DETAILLEE DE L'INVENTION

Les références aux méthodes de traitement dans la présente description doivent être interprétées comme des références aux combinaisons et compositions selon la présente invention destinées à être utilisées dans une méthode de traitement d'un sujet par thérapie.

L'endométriose est une maladie caractérisée par la présence d'endomètre ectopique qui forme des lésions kystiques sur les ovaires et dans la cavité péritonéale. La physiopathologie de cette maladie est peu connue. Des études récentes amènent à penser que l'endométriose serait une maladie immunologique, inflammatoire estrogène-dépendante voire auto-immune (Ahn, 2016 ; Khan, 2010 ; Lin, 2016 ; Malutan, 2015). Il a été également évoqué qu'une dysbiose et/ou une hyperperméabilité intestinale et vaginale ainsi que le stress oxydatif auraient un impact sur la progression de la maladie (Baker, 2017 ; Laschke, 2015 ; Puca, 2017 ; Ito, 2017 ; Scutiero, 2017.

Les Inventeurs ont montré de manière surprenante que l'administration combinée d'une levure *Saccharomyces cerevisiae,* de resvératrol et d'un extrait de curcuma (*Curcuma longa*) permettait de ralentir de manière significative la progression des lésions d'endométriose chez un modèle animal d'endométriose, par rapport aux animaux témoins et aux animaux traités par une combinaison de resvératrol et d'un extrait de curcuma et ceci au bout de 12 semaines de traitements.

Les Inventeurs ont également montré que cette combinaison d'actifs permettait de moduler le statut immunitaire en augmentant la proportion de macrophages de type 1 par rapport aux macrophages de type 2. Un tel effet n'a pas été observé chez les animaux témoins ou traités par la combinaison de resvératrol et d'un extrait de curcuma.

Ainsi, les Inventeurs ont montré que les trois ingrédients actifs selon l'invention, à savoir la levure *Saccharomyces cerevisiae,* le resvératrol et l'extrait de curcuma, exercent une action synergique permettant de stopper voire de faire régresser les lésions d'endométriose et de moduler le statut immunitaire chez le patient malade.

Les Inventeurs ont également montré que l'administration combinée de resvératrol, de l'extrait de curcuma et d'une levure *Saccharomyces cerevisiae* permettait de diminuer la production de cytokines pro-inflammatoires telles que le TNFα et l'interleukine 6, ainsi que le stress oxydatif cellulaire. De manière notable, cette combinaison d'actifs a permis également de diminuer le taux plasmatique de zonuline, une protéine impliquée dans l'hyperperméabilité intestinale, biomarqueur de réactions inflammatoires de bas grade.

En résumé, l'administration combinée de resvératrol, d'un extrait de curcuma et d'une levure *Saccharomyces cerevisiae* permet de prévenir voire de traiter, le développement, des lésions d'endométriose. Cette combinaison d'actifs permet également de moduler le système immunitaire, notamment d'orienter le rapport des macrophages M1/M2, et de normaliser le statut inflammatoire au niveau péritonéal chez le sujet souffrant d'endométriose, notamment en diminuant le stress oxydatif et la perméabilité intestinale.

Ainsi, l'invention a pour objet l'utilisation du resvératrol, d'un extrait de curcuma comprenant au moins 15% en poids de curcuminoïdes et de levure *Saccharomyces cerevisiae* de variété boulardii en combinaison pour traiter un sujet souffrant d'endométriose ou susceptible de souffrir d'endométriose. Plus précisément, l'invention concerne l'utilisation du resvératrol, d'un extrait de curcuma comprenant au moins 15% en poids de curcuminoïdes et de levure *Saccharomyces cerevisiae* de variété boulardii en combinaison dans le traitement ou la prévention de l'endométriose ou de l'un de ses symptômes ou troubles associés.

Il est également décrit une méthode de traitement d'un sujet souffrant ou susceptible de souffrir d'endométriose comprenant l'administration d'une dose efficace de resvératrol, d'un extrait de curcuma et de levure *Saccharomyces cerevisiae* audit sujet. L'invention a également pour objet un kit ou une composition, de préférence, pharmaceutique, nutraceutique ou alimentaire, comprenant l'association de resvératrol, d'un extrait de curcuma comprenant au moins 15% en poids de curcuminoïdes et de levure *Saccharomyces cerevisiae* de variété boulardii ladite composition ou ledit kit étant adapté(e) aux sujets souffrant ou susceptibles de souffrir d'endométriose.

### Définitions générales

Tel qu'utilisé ici, les termes « *désordre* », « *anomalie* » ou « *trouble* » sont interchangeables et font référence à un organe, une partie, une structure ou un système du corps qui fonctionne incorrectement. De préférence, le terme « *trouble* » désigne un trouble de la santé, par exemple perturbant les fonctions physiques ou mentales normales.

Telle qu'utilisée ici, une « *composition pharmaceutique* » désigne une préparation d'un ou plusieurs des agents actifs avec d'autres composants chimiques optionnels tels que des supports physiologiquement appropriés et/ou des excipients. Le but d'une composition pharmaceutique est de faciliter l'administration de l'agent actif à un organisme. Les compositions de la présente invention peuvent se présenter sous une forme appropriée pour toute voie d'administration ou d'utilisation conventionnelle. La composition pharmaceutique comprend généralement un véhicule pharmaceutiquement acceptable.

Tel qu'utilisé ici, les termes « *support pharmaceutiquement acceptable* » ou « *excipient pharmaceutiquement acceptable* » ou « *véhicule pharmaceutiquement acceptable* » sont interchangeables et comprennent tous composés ou combinaisons de composés qui sont connus de l'homme de l'art comme étant utiles dans la formulation de compositions pharmaceutiques ou vétérinaires. Dans le contexte de la présente invention, on entend par « physiologiquement acceptable » ou « pharmaceutiquement acceptable », tout milieu ou additif qui n'interfère pas avec l'efficacité de l'activité biologique du principe actif (ici, la combinaison de resvératrol, d'extrait de curcuma et de levure *S. cerevisiae*), et qui n'est pas excessivement toxique pour le sujet, aux concentrations auxquelles il est administré et/ou qui ne produit pas de réaction indésirable lorsqu'il est administré. Un véhicule, un support ou un excipient physiologiquement acceptable peut être approprié à l'administration aux humains et/ou aux animaux (en particulier aux mammifères).

Le terme « *médicament* », tel qu'utilisé ici, englobe des médicaments à usage humain en médecine humaine et fait référence à toute substance acceptable sur le plan pharmacologique qui procure un effet thérapeutique et/ou bénéfique. Le terme « médicament » utilisé ici n'est pas nécessairement limité aux substances nécessitant une autorisation de mise sur le marché, mais peut comprendre des substances qui peuvent être utilisées dans les cosmétiques et les compositions nutraceutiques, par exemple les compléments alimentaires.

Le terme *« nutraceutique* » fait référence à une composition ou produit fabriqué à partir de substances alimentaires, mais rendu disponible sous forme de comprimé, de poudre, de potion ou d'autre formes galéniques habituellement non associées à des aliments, et ayant un effet physiologique bénéfique ou protecteur contre des troubles ou maladies humaines. On retrouve notamment sous cette définition les compléments alimentaires, certains aliments pour groupe spécifique ou les substituts de repas.

On entend par « *composition nutraceutique* » toute composition comprenant des ingrédients alimentaires tels que des macronutriments, des micronutriments, des plantes ou extraits de plantes, ou les substances ayant un effet nutritionnel ou physiologique, visant à compléter le régime alimentaire d'un être humain afin d'améliorer le statut nutritionnel de ce dernier et favoriser ainsi un bon état de santé. Tels qu'envisagés ici, les produits nutraceutiques sont des nutriments isolés, des compléments alimentaires ou sont inclus dans certains aliments destinés à des groupes spécifiques. Les compositions nutraceutiques englobent ainsi par exemple la combinaison selon l'invention et, en option, un composant de qualité alimentaire, en particulier un additif ou auxiliaire technologique.

Par « *complément alimentaire* », est entendu ici toute composition qui est formulée et administrée séparément d'autres aliments et a pour but de complémenter les apports nutritionnels d'un sujet ayant une forme acceptable sur le plan pharmacologique, notamment sous forme de gélules, comprimés, capsules molles, sachets, stick-packs, sirop, solution buvable conditionnée en ampoule, compte-gouttes, ou toute autre forme adaptée bien connue de l'homme du métier.

Les termes « *aliment* », « *produit alimentaire* » et « *denrée alimentaire* » sont utilisés de manière interchangeable ici et comprennent, en plus des aliments couramment consommés par les humains, les aliments fonctionnels et les aliments pour groupes spécifiques, eux même comprenant les denrées destinées à des fins médicales spéciales.

On entend par « *composition alimentaire* » toute composition comprenant des ingrédients alimentaires tels que des macronutriments, des micronutriments, des vitamines, des minéraux, oligo-éléments ou des substances ayant un effet nutritionnel ou physiologique. Tels qu'envisagées ici, les produits alimentaires sont des aliments conventionnels, des aliments fonctionnels ou des aliments transformés. Ainsi, par définition, les compositions alimentaires peuvent être des aliments divers et variés, connus de l'homme du métier.

La composition alimentaire peut être notamment un aliment destiné à l'alimentation humaine qui pourra être un liquide, une pâte ou un solide. A titre d'exemples, sans s'y limiter, l'aliment pourra être un produit laitier tel que du fromage, du beurre, une crème, les yaourts, les glaces, les fromages, les produits cuits tels que le pain, les biscuits et les gâteaux, un produit à base de fruit comme un jus de fruit, une compote ou une pâte de fruit, les produits alimentaires à base de soja, les produits alimentaires à base d'amidon, produits de confiserie, compositions pour huiles comestibles, pâtes à tartiner, céréales pour petit déjeuner, les barres alimentaires; biscuits, snack, chewing-gums; des boissons; boissons énergisantes, boissons enrichies; suppléments à boire (des poudres à ajouter à une boisson); etc.

Selon un aspect particulier, la composition alimentaire n'est pas à base de lait. De préférence, la composition alimentaire ne comprend pas de protéine(s) ou d'oligosaccharide(s) du lait humain ou animal. Par exemple, la composition alimentaire selon l'invention comprend la combinaison d'actifs selon l'invention et les composants de l'un au moins des aliments ou des boissons précédemment cités.

Le terme « *aliment fonctionnel* » fait référence ici à un aliment conventionnel faisant partie de l'alimentation normale et procurant des bienfaits physiologiques et/ou réduisant le risque de maladies ou de troubles et/ou réduisant les symptômes y étant associés, au-delà des nutriments traditionnels qu'il contient.

Tel qu'utilisé ici, le terme « *additif alimentaire* » se réfère à un composé ou une composition qui est destiné à être mélangé avec un ou plusieurs autres aliments avant d'être administré à un sujet. Plus particulièrement, on entend par «*additif alimentaire*» tout additif tel que défini par le Codex alimentarius, Norme générale pour les additifs alimentaires - Codex Stan 192-1995, c'est à dire toute substance qui n'est pas consommée seule en tant que denrée alimentaire, ni utilisée seule comme ingrédient caractéristique d'une denrée alimentaire, qu'elle ait ou non une valeur nutritive, et dont l'addition intentionnelle à une denrée alimentaire dans un but technologique (y compris organoleptique) à une étape quelconque de la fabrication, de la transformation, de la préparation, du traitement, du conditionnement, de l'emballage, du transport ou de l'entreposage de ladite denrée entraîne, ou peut, selon toute vraisemblance, entraîner, directement ou indirectement, son incorporation ou celle de ses dérivés dans cette denrée ou en affecter d'une autre façon les caractéristiques.

Tel qu'utilisé ici, un *« prébiotique* » se réfère à un ingrédient ou substrat qui, utilisé sélectivement par un microorganisme de l'hôte, confère un bénéfice sur la santé. Il peut permettre d'induire des changements bénéfiques, à la fois dans la composition et/ou l'activité d'un probiotique. Un prébiotique peut être un aliment ou une boisson comestible ou un ingrédient de ceux-ci. Les prébiotiques peuvent inclure des hydrates de carbones complexes, des polyphénols et des acides gras polyinsaturés. Un prébiotique est habituellement un glucide non digestible tel qu'un oligo- ou polysaccharide, ou un alcool de sucre, qui n'est pas dégradé ou absorbé dans le tube digestif supérieur en l'absence de micro-organismes intestinaux. De préférence, les prébiotiques selon l'invention sont tels que définis dans l'Arrêté du 26 septembre 2016 établissant la liste des substances à but nutritionnel ou physiologique autorisées dans les compléments alimentaires et les conditions de leur emploi.

Tel qu'utilisé ici, le terme « *probiotique* » se réfère à des bactéries vivantes ou inactivées qui, lorsqu'elles sont administrées en quantités adéquates, ont un effet bénéfique sur l'organisme hôte. Ces compositions sont avantageuses en ce qu'elles sont appropriées pour une administration aux humains et à d'autres sujets mammifères. Les substances probiotiques contiennent un nombre suffisamment élevé de micro-organismes probiotiques pour exercer une action directe ou indirecte sur le microbiote intestinale. Il est à noter que, aux fins de la présente description, on entend par probiotique toute forme biologiquement active de probiotique, de préférence mais non limitativement, les levures, les lactobacilles, bifidobactéries, bacillus, streptocoques, entérocoques, propionibactéries ou saccaromycètes mais encore d'autres microorganismes constituant la « flore intestinale normale », ou encore des fragments de la paroi bactérienne ou de l'ADN de ces microorganismes. De préférence, les probiotiques selon l'invention sont tels que définis par l'Organisation des Nations Unies pour l'alimentation et l'agriculture (FAO) et l'Organisation Mondiale de la Santé (OMS) dans la Consultation mixte d'experts FAO/OMS sur l'évaluation des propriétés sanitaires et nutritionnelles des probiotiques dans les aliments, y compris le lait en poudre contenant des bactéries lactiques vivantes réalisée en 2001.

Les termes « microbiote » ou « microflore » utilisés ci-après sont interchangeables et désignent l'ensemble des espèces microbiennes présentes (de manière durable ou transitoire) dans un environnement, comprenant des eucaryotes, des archées, des procaryotes et des virus. Le terme « microbiome » fait référence au « contenu génétique » du microbiote et comprend l'ADN génomique, l'ARN ribosomique, l'épigénome, les plasmides et tous les autres types d'informations génétiques du microbiote. Préférentiellement, ces termes font référence ici à des bactéries du microbiote intestinal humain.

Tel qu'utilisé ici, le terme « *levure* » désigne des champignons microscopiques, aux cellules ovoïdes de quelques millièmes de millimètres de largeur, se détachant facilement les unes des autres et, de ce fait, bien adaptées à la propagation dans les liquides. Le terme « levure » englobe toutes les espèces du genre *Saccharomyces.*

Tel qu'utilisé ici, le terme « *bactérie* » désigne tout micro-organisme procaryote existant sous la forme d'une cellule unique, dans un groupe ou dans un agrégat de cellules individuelles. Le terme « *bactérie* » englobe toutes les variantes de bactéries (par exemple, les bactéries endogènes ou les bactéries environnementales), plus particulièrement les bactéries du microbiote humain ou animal, notamment le microbiote intestinal.

Tel qu'utilisé ici, le terme « *comprenant* » ou « *comprend* » est utilisé en référence à des substances, composés ou procédés qui sont essentiels à l'invention, mais qui sont ouverts à l'inclusion d'éléments non spécifiques, essentiels ou non. L'utilisation de « comprenant » indique l'inclusion plutôt que la limitation.

Le terme « *et*/*ou* » tel qu'il est utilisé ici doit être considéré comme une description spécifique de chacune des deux caractéristiques ou composants spécifiés, avec ou sans l'autre. Par exemple, « A et/ou B » doit être considéré comme une divulgation spécifique de chacun des éléments suivants : (i) A, (ii) B et (iii) A et B, comme si chacun d'eux était présenté individuellement.

Le terme « *environ* » tel qu'utilisé ici en relation avec toutes les valeurs (incluant les extrémités inférieures et supérieures de plages numériques) signifie toute valeur ayant une variation acceptable allant jusqu'à +/- 10% (par exemple, +/- 0,5% , +/- 1%, +/- 1,5%, +/- 2%, +/- 2,5%, +/- 3%, +/- 3,5%, +/- 4%, +/- 4,5% , +/- 5%, +/- 5,5%, +/- 6%, +/- 6,5%, +/- 7%, +/- 7,5%, +/-8%, +/- 8,5%, + / - 9%, +/- 9,5%). L'utilisation du terme "environ" au début d'une liste de valeurs modifie chacune d'entre elles (c'est-à-dire « environ 1, 2 et 3 » se réfère à environ 1, environ 2 et environ 3). En outre, lorsqu'une liste de valeurs est décrite (par exemple environ 50%, 60%, 70%, 80%, 85% ou 86%), la liste inclut toutes ses valeurs intermédiaires et fractionnaires (par exemple 54% ou 85,4%).

Le terme « *consiste essentiellement en* » tel qu'utilisé ici pour définir une composition dans laquelle les éléments autres que ceux-mentionnés ne représentent pas plus 5% en poids de la composition, de préférence pas plus de 3% en poids, et de manière plus spécifique pas plus de 1 % en poids.

### Utilisations selon l'invention

Selon un premier aspect, l'invention a pour objet l'utilisation du resvératrol, d'un extrait de curcuma comprenant au moins 15% en poids de curcuminoïdes et de levure *Saccharomyces cerevisiae* de variété Boulardii en combinaison dans la prise en charge d'un sujet souffrant ou susceptible de souffrir d'endométriose.

La combinaison selon l'invention peut être utilisée pour traiter un sujet souffrant d'endométriose. Plus précisément, l'invention concerne l'utilisation du resvératrol, d'un extrait de curcuma comprenant au moins 15% en poids de curcuminoïdes et de levure *Saccharomyces cerevisiae* de variété Boulardii en combinaison dans le traitement ou la prévention de l'endométriose ou de l'un de ses symptômes ou troubles associés.

L'invention a notamment pour objet l'utilisation d'une combinaison de resvératrol, d'un extrait de curcuma comprenant au moins 15% en poids de curcuminoïdes et de levure *Saccharomyces cerevisiae* de variété Boulardii pour traiter ou prévenir les lésions d'endométriose. Plus particulièrement, la combinaison selon l'invention peut être utilisée pour prévenir ou ralentir le développement de lésions d'endométriose, ou pour diminuer le volume ou le poids de lésions d'endométriose chez un sujet souffrant d'endométriose ou chez un sujet susceptible de souffrir d'endométriose.

Selon un certain aspect, la combinaison de resvératrol, d'un extrait de curcuma comprenant au moins 15% en poids de curcuminoïdes et de levure *Saccharomyces cerevisiae* de variété Boulardii selon l'invention est utilisée pour prévenir ou traiter, e.g. diminuer, l'inflammation péritonéale chronique chez un sujet souffrant ou susceptible de souffrir d'endométriose.

Selon un aspect supplémentaire, la combinaison de resvératrol, d'un extrait de curcuma comprenant au moins 15% en poids de curcuminoïdes et de levure *Saccharomyces cerevisiae* de variété Boulardii selon l'invention est utilisée pour prévenir ou traiter, e.g. diminuer l'hyperperméabilité intestinale, ou pour normaliser la perméabilité intestinale chez un sujet souffrant ou susceptible de souffrir d'endométriose.

Selon un autre aspect, la combinaison de resvératrol, d'un extrait de curcuma et de levure *Saccharomyces cerevisiae* de variété Boulardii selon l'invention est utilisée pour normaliser ou moduler le statut immunitaire, e.g. promouvoir un fonctionnement normal du système immunitaire, chez un sujet souffrant ou susceptible de souffrir d'endométriose.

Selon un aspect supplémentaire, la combinaison de resvératrol, d'un extrait de curcuma comprenant au moins 15% en poids de curcuminoïdes et de levure *Saccharomyces cerevisiae* de variété Boulardii selon l'invention est utilisée pour prévenir ou diminuer le stress oxydatif au niveau péritonéal chez un sujet souffrant ou susceptible de souffrir d'endométriose.

Selon un autre aspect, la combinaison de resvératrol, d'un extrait de curcuma comprenant au moins 15% en poids de curcuminoïdes et de levure *Saccharomyces cerevisiae* de variété Boulardii selon l'invention est utilisée pour prévenir ou traiter un ou plusieurs symptômes ou troubles associés à l'endométriose.

La combinaison selon l'invention peut être, par exemple, utilisée pour prévenir ou diminuer la fréquence et/ou l'intensité des douleurs comme les douleurs pelviennes telles que les dysménorrhées et les dyspareunies et les douleurs cataméniales par exemple au niveau intestinale ou urinaire chez un sujet souffrant ou susceptible de souffrir d'endométriose.

La combinaison selon l'invention peut être également utilisée pour traiter ou prévenir l'hypofertilité ou l'infertilité, chez un sujet souffrant ou susceptible de souffrir d'endométriose. La combinaison selon l'invention peut être également utilisée pour améliorer la fertilité, ou maintenir une fertilité normale, chez un sujet souffrant ou susceptible de souffrir d'endométriose.

La combinaison selon l'invention peut être utilisée pour maintenir la fertilité chez un sujet souffrant d'endométriose ou chez un sujet susceptible de souffrir d'endométriose. La combinaison selon l'invention peut être notamment utilisée pour normaliser or améliorer le statut hormonal d'un sujet souffrant d'endométriose ou chez un sujet susceptible de souffrir d'endométriose, par exemple normaliser ou améliorer le taux plasmatique d'AMH (Hormone Anti-müllérienne) et/ou d'oestradiol.

Selon un aspect supplémentaire, la combinaison selon l'invention peut être utilisée pour améliorer la condition générale d'un sujet souffrant ou susceptible de souffrir d'endométriose. On entend par « *améliorer la condition générale* » le fait d'améliorer l'état physiologique et/ou psychologique d'un sujet, ce qui peut conduire notamment à une amélioration de sa qualité de vie. La combinaison selon l'invention peut rendre plus supportable la maladie et les symptômes associés. Il peut s'agir d'une utilisation non-thérapeutique de la combinaison selon l'invention. Ainsi, selon un aspect supplémentaire, l'invention a pour objet l'utilisation non-thérapeutique d'une combinaison de resvératrol, d'un extrait de curcuma et de levure *Saccharomyces cerevisiae* en tant qu'agent pour améliorer le bien-être et/ou la qualité de vie d'un sujet ayant ou susceptible de souffrir de l'endométriose. L'invention a notamment pour objet l'utilisation non-thérapeutique d'une combinaison de resvératrol, d'un extrait de curcuma et de levure *Saccharomyces cerevisiae* en tant que complément alimentaire dans la prise en charge d'un suj et ayant ou susceptible de souffrir de l'endométriose.

### 1. L'endométriose et symptômes et troubles associés, sujet selon l'invention

Comme indiqué dans la partie arrière-plan technologique, l'endométriose est une pathologie se caractérisant par la présence de tissu endométrial en dehors de la cavité utérine. Ce tissu peut se retrouver notamment au niveau des parois de l'utérus, des trompes de Fallope, des ovaires, des ligaments utérosacrés mais aussi au niveau de la vessie et du côlon.

Au sens de l'invention, le terme « *endométriose* » englobe l'ensemble des formes d'endométriose connues, y compris l'adénomyose, les endométriomes, l'endométriose profonde ou encore les endométrioses de stades I à IV selon la classification de l'American Fertility Society.

Au sens de l'invention, les symptômes et troubles associés à l'endométriose englobent, sans y être limités, les douleurs, les saignements anormaux ou excessifs tels que les rectorragies, le sang dans les urines et les métrorragies, les troubles digestifs ou urinaires tels que des diarrhées, la constipation, les mictions fréquentes et/ou douloureuses notamment au moment des règles, la fatigue chronique, l'hypofertilité et l'infertilité. Les symptômes et troubles associés à l'endométriose englobent également une inflammation chronique péritonéale, une hyperperméabilité intestinale, et une dysbiose vaginale et/ou intestinale.

Les douleurs associées à l'endométriose sont diverses et englobent notamment les dysménorrhées (douleurs pelviennes pendant les règles), les douleurs lombaires, les dyspareunies et les douleurs cataméniales notamment au niveau urinaire ou intestinale.

Dans le cadre de la présente invention, on entend par « *hypofertilité* » le fait qu'une femme ne soit pas parvenue à tomber enceinte après au moins 6 mois de rapports sexuels réguliers sans contraception. On entend par « *infertilité* » le fait qu'une femme ne soit pas parvenue à tomber enceinte après 24 mois de rapports sexuels réguliers sans contraception.

Des « *rapports sexuels réguliers* » correspond à environ au moins un rapport sexuel toute les 48h.

Dans un mode de réalisation particulier, la combinaison d'actifs selon l'invention est utilisée pour traiter ou prévenir un symptôme ou trouble associé à l'endométriose choisi parmi le groupe constitué par les dysménorrhées, les dyspareunies, la fatigue chronique, l'hypofertilité l'infertilité et les combinaisons de ceux-ci.

Dans le cadre de l'invention, on entend par le terme de « *traiter, traitement* » l'obtention d'un effet pharmacologique, et/ou physiologique souhaité. L'effet peut être prophylactique en termes de prévention totale ou partielle d'une pathologie ou de l'un de ses troubles ou symptômes associés et/ou peut être thérapeutique en termes de guérison partielle ou complète de ladite maladie. Le terme « traitement » tel qu'utilisé ici couvre tout traitement d'une maladie chez un sujet d'intérêt, pour : réduire l'incidence et/ou le risque de rechute de la maladie ou du trouble pendant une période sans symptômes; soulager ou réduire un symptôme ou un trouble associé à la maladie; empêcher la maladie ou l'un de ses symptômes ou troubles associés ou le trouble de se produire chez un sujet malade ou prédisposé à la maladie; stopper, ralentir ou décaler dans le temps le développement de la maladie ou de l'un de ses symptômes ou troubles associés; réduire la fréquence et/ou l'intensité des épisode de la maladie, par exemple des symptômes; et soulager la maladie ou un symptôme ou trouble associé, c'est-à-dire provoquer une régression totale ou partielle de ladite maladie, symptôme ou trouble associé.

A titre d'exemple, la combinaison selon l'invention peut être utilisée pour prévenir (e.g. empêcher, ralentir, ou décaler dans le temps) l'apparition et le développement de lésions d'endométriose et/ou diminuer, voire traiter, les lésions d'endométriose.

A titre d'exemple supplémentaire, la combinaison selon l'invention peut être utilisée pour traiter, e.g. diminuer la fréquence et/ou l'intensité des douleurs pelviennes chez une femme souffrant d'endométriose.

Le sujet selon l'invention est une femme menstruée, de tout âge notamment entre 12 ans et 50 ans, de préférence entre 14 ans et 45 ans.

On entend par un sujet souffrant d'endométriose une femme pour laquelle la présence d'au moins une lésion d'endométriose a été diagnostiquée, par exemple lors d'un examen clinique, par une technique d'imagerie (échographie, IRM) ou par coelioscopie. Une femme souffrant d'endométriose englobe notamment une femme en rémission, par exemple une femme ayant subi un traitement médical ou chirurgical visant à éliminer ou diminuer les lésions d'endométriose.

Le sujet souffrant d'endométriose peut être sous un traitement de prise en charge de l'endométriose ou l'un de ses symptômes, incluant la prise de contraceptifs, notamment oraux, les AINS, et les traitements utilisés en procréation médicalement assistée, notamment les traitements de stimulation ovarienne, ou encore un traitement de cure thermale. Il peut s'agir également d'un traitement par médecine douce (ostéopathie, acupuncture, kinésithérapie, autohypnose, sophrologie...)

Une femme souffrant d'endométriose peut présenter un ou plusieurs troubles ou symptômes décrits ci-avant. Dans un mode de réalisation préféré, une femme souffrant d'endométriose présente au moins un symptôme ou un trouble choisi parmi les dysménorrhées, les dyspareunies, la fatigue chronique, l'infertilité et les combinaisons de ceux-ci.

On entend par « *un sujet susceptible de souffrir d'endométriose* » ou « *un sujet prédisposé à l'endométriose* » une femme ayant un tableau clinique évoquant la présence d'endométriose, par exemple présentant des douleurs fortes voire intenses pendant ses règles, et/ou une hypofertilité ou infertilité et/ou ayant des antécédents familiaux (par exemple une mère, une tante, une grand-mère et/ou une soeur souffrant d'endométriose).

### 2. Combinaison d'ingrédients actifs selon l'invention

La combinaison d'ingrédients actifs selon l'invention comprend du resvératrol, un extrait de curcuma et une levure *Saccharomyces cerevisiae.*

Selon l'invention, on entend par « *combinaison d'ingrédients actifs* » ou encore « *association d'ingrédients actifs* » un ensemble d'actifs pouvant agir de manière coopérative ou synergique afin d'obtenir un ou plusieurs effets pharmacologiques, et/ou physiologiques et/ou nutritionnels visant à améliorer l'état général d'un sujet et/ou traiter ou prévenir une pathologie ou l'un de ses symptômes ou troubles associés. Comme expliqué ci-avant, la combinaison d'ingrédients actifs selon l'invention trouve une application dans la prise en charge des sujets souffrant ou susceptibles de souffrir d'endométriose.

### Le resvératrol

Le resvératrol est un polyphénol de la classe des stilbènes présent dans de nombreuses espèces végétales. Au sens de l'invention, le terme resvératrol englobe à la fois l'isomère trans et l'isomère cis. Dans certains modes de réalisations, le resvératrol est sous forme d'un mélange d'isomères trans- et cis. Dans d'autres modes de réalisation, le resvératrol est sous forme d'isomère trans. Il s'agit donc du trans-resvératrol de n° de CAS 501-36-0.

De préférence, le resvératrol est présent sous forme de trans-resvératrol.

Le resvératrol peut être préparé par synthèse chimique ou purifié à partir de toute plante ou partie de plante connue comme comprenant du resvératrol comme les raisins, pépins, feuilles et sarments, de vigne, la renouée du Japon, le cacao, la canneberge, les mûres ou encore les noix.

Le resvératrol peut être apporté sous forme pure c'est-à-dire présentant une pureté d'au moins 99%.

Le cas échéant, le resvératrol peut être apporté sous la forme d'un extrait végétal enrichi en resvératrol, c'est-à-dire présentant au moins 50% (e.g. au moins 60%, 70%, 80%, 85%, 90% ou 95%) en poids de resvératrol par rapport au poids de matière sèche de l'extrait.

Dans un mode de réalisation particulier, le resvératrol est présent sous la forme d'un extrait de renouée du Japon (*Fallopia japonica (Houtt.) Ronse Dec., Polygonaceae*) titré à au moins 95%, e.g. au moins 98% en poids de trans-resvératrol par rapport au poids total de la composition.

Selon l'invention, la dose journalière de resvératrol peut être comprise de 10 mg à 300 mg, par exemple de 25 mg à 250 mg, de 25 mg à 100 mg, de préférence de 30 mg à 70 mg ou de 40 mg à 60 mg.

### La levure Saccharomyces cerevisiae

Au sens de l'invention, on entend par « levure *Saccharomyces cerevisiae »* n'importe quelle levure appartenant à l'espèce *Saccharomyces cerevisiae* de variété boulardii.

Ainsi, l'invention est mise en oeuvre avec une levure *Saccharomyces cerevisiae* de variété boulardii.

La levure *Saccharomyces cerevisiae* est ainsi choisie dans le groupe constitué par les souches de *Saccharomyces cerevisiae* var. boulardii,.

Plusieurs souches de *Saccharomyces cerevisiae* de variété boulardii, sont disponibles dans le commerce.

On peut citer notamment la souche commercialisée par Lesaffre Human Care sous la marque Lynside^{®} Pro SCB et enregistrée sous le numéro CNCM (Collection Nationale de Cultures de Microorganismes) I-3799.

A titre d'exemple supplémentaire, on peut aussi citer la souche de numéro CNCM I-745 déposée le 28 mars 1988 auprès de la CNCM-Institut Pasteur par les Laboratoires Biocodex et disponible commercialement, par exemple dans les produits Ultra-levure^{®}. On peut également citer la souche de numéro CNCM I-1079 commercialisée par la société Lallemand ou encore les souches déposées sous le n°ATCC (American Type Culture Collection) MYA-796 ou MYA-797.

Il est entendu que la présente invention concerne également l'utilisation de toute souche issue de l'espèce *Saccharomyces cerevisiae*de variété boulardii ainsi que l'utilisation de tout homologues issu de celle-ci. Les termes « *homologue* », « *variant* » ou « *mutant* » sont interchangeables et réfèrent à une souche bactérienne ayant une homologie ou une identité de séquence avec la séquence nucléotidique de la souche bactérienne parente (la séquence de référence). Ceci inclut des souches ayant au moins 95%, 96%, 97%, 98% ou 99% d'identité avec la séquence nucléotidique du génome de la souche *Saccharomyces cerevisiae var. boulardii* enregistrée sous le numéro CNCM I-745 ou celle enregistrée sous le numéro CNCM I-3799. Les mutants peuvent être obtenus par des techniques de génie génétique permettant d'inférer l'altération du matériel génétique des souches de l'invention ou d'inférer une recombinaison du matériel génétique des souches de l'invention avec d'autres molécules. Typiquement, afin d'obtenir de telles souches mutantes, l'homme du métier peut utiliser des techniques de mutagenèse standard telles qu'un rayonnement UV ou une exposition à des produits chimiques mutagènes.

La levure *Saccharomyces cerevisiae* présente dans la combinaison selon l'invention peut être vivante ou inactivée. De préférence, la levure est sous forme vivante. La levure *Saccharomyces cerevisiae* peut notamment être sous une forme hydratée, lyophilisée, congelée, atomisée ou toute autre forme lui permettant d'être administrée vivante au sujet. La levure peut être administrée telle quelle ou après reprise dans un véhicule physiologiquement acceptable approprié.

Les levures sont administrées, par exemple, sous la forme de bactéries vivantes cultivées, sous forme végétative ou sous forme de spores. Alternativement, les levures sont fournies sous forme de populations purifiées obtenues à partir d'un matériau microbiotique tel qu'un matériau fécal. Par vivante, il faut comprendre que l'intégrité de la cellule est conservée et que les processus cellulaires se produisent ou peuvent se produire si la levure est cultivée dans un milieu et des conditions adéquates. Toute levure vivante peut être de nouveau ensemencée sur un milieu de culture adéquat et se multiplier, dans des conditions adéquates.

Dans certains modes de réalisation, la levure *Saccharomyces cerevisiae* est administrée à une dose de 10⁷ à 10¹¹, notamment de 10⁷ à 10¹⁰, de préférence de 10⁸ à 10¹⁰ ou de 10⁸ à 10⁹ unité formant des colonies (CFU).

A titre d'exemple, la levure *Saccharomyces cerevisiae* est administrée en une dose en masse allant de 5 à 100 mg.

Il s'agit d'une souche appartenant à la variété *Saccharomyces cerevisiae* var. boulardii, par exemple celle déposée sous le N°CNCM I-3799 ou I-745.

De manière préférée, la combinaison selon l'invention comprend l'administration journalière de 2×10⁸ à 6×10⁸ CFU, de manière plus préférée de 3×10⁸ à 5×10⁸ CFU de *Saccharomyces cerevisiae* var. boulardii,.

### L'extrait de curcuma

Selon l'invention, le *Curcuma longa L.*, encore appelé curcumin, curcuma ou curcumine, est une plante vivace originaire d'Asie qui appartient à la famille des Zingiberacées. Le rhizome de curcuma est constitué de mucilages, de polysaccharides, d'huiles essentielles, de polyphénols, et de curcuminoïdes, donnant notamment la couleur jaune /orange au rhizome.

On entend par « *curcuminoïdes* » des composés de type diarylheptanoïdes comprenant notamment la curcumine et ses dérivés telles que la déméthoxycurcumine ou encore la bis-démethoxycurcumine.

La curcumine (1,7-bis(4-hydroxy-3-méthoxyphényl)-1,6-heptadiène-3,5-dione), également appelée diféruloylméthane (N° CAS : 458-37-7), est le curcuminoïde principal du curcuma asiatique.

L'extrait de curcuma présent dans la combinaison est typiquement un extrait de rhizome, sec ou frais, *Curcuma longa L.* Il peut s'agir d'un extrait standardisé en curcuminoïdes.

Au sens de l'invention, on entend par le terme « *standardisé* » ou « *standardiser* », la notion de contrôle d'un extrait, d'une huile ou tout autre ingrédient afin de le rendre conforme à une norme ou à une teneur minimale définie en certaines molécules, comme par exemple les curcuminoïdes dans le cas de l'extrait de curcuma. Un extrait de curcuma standardisé en curcuminoïdes est donc un extrait de curcuma présentant une teneur minimale définie en curcuminoïdes, de préférence une teneur minimale de 15% en curcuminoïdes.

Ainsi, l'extrait de curcuma selon l'invention comprend une teneur d'au moins 15% en poids de curcuminoïdes par rapport au poids total de l'extrait, de préférence par rapport au poids total de matière sèche de l'extrait.

L'extrait de curcuma présent dans la combinaison selon l'invention peut être obtenu par toute méthode d'extraction connue par exemple par extraction à l'aide d'un solvant organique, par macération ou décoction ou encore par extraction par fluide supercritique. De préférence il s'agit d'un extrait de rhizome de curcuma obtenu par un procédé comprenant une étape d'extraction avec un solvant utilisable dans la préparation de compléments alimentaires, par exemple un alcool ou un ester de type acétate d'éthyle.

De manière avantageuse, l'extrait de curcuma selon l'invention est sous forme de poudre. Sa teneur en eau est de préférence inférieure à 15% (e.g. inférieure à 10% ou 5%) par rapport au poids total de l'extrait. Selon un mode préféré de réalisation de l'invention, l'extrait de curcuma contient au moins 10%, 15%, 20%, 25%, 30%, 35%, 40% ou 45% en poids de curcuminoïdes. Dans un mode particulier de réalisation, l'extrait de curcuma comprend de la curcumine, de la déméthoxycurcumine et/ou de la bisdéméthoxycurcumine. De préférence, la curcumine est le curcuminoide majoritaire de l'extrait. La curcumine peut représenter au moins 55%, de préférence au moins 60% en poids par rapport au poids total des curcuminoïdes présents dans l'extrait.

A titre d'exemple, l'extrait de curcuma comprend de la curcumine, de la déméthoxycurcumine et de la bisdéméthoxycurcumine selon les rapports suivants : de 65 à 82% de curcumine, de 15 à 25 % de déméthoxycurcumine et de 2 à 7% de bisdéméthoxycurcumine, les pourcentages étant exprimés en poids par rapport au poids total de curcuminoïdes présents dans l'extrait.

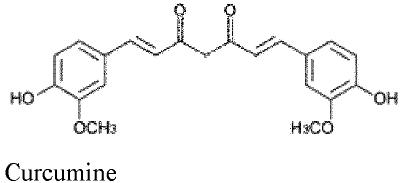

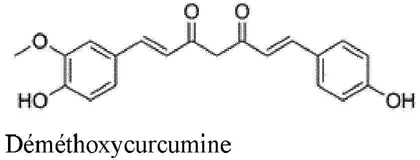

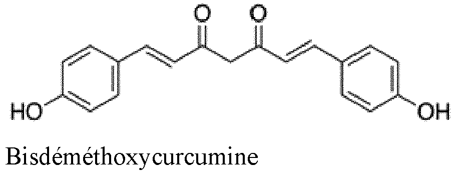

Afin d'améliorer la biodisponibilité des curcuminoïdes, l'extrait de curcuma peut être formulé à l'aide d'un vecteur ou d'un système d'encapsulation tels que des liposomes ou des molécules cages. Dans un mode de réalisation particulier, l'extrait de curcuma est associé à une cyclodextrine, de préférence une gamma-cyclodextrine. Il peut s'agir notamment du produit CAVACURMIN^{®} commercialisé par Wacker.

Selon un aspect particulier, la combinaison selon l'invention comprend de la curcumine introduite dans la composition par l'ajout d'un extrait de curcuma ou sous une forme purifiée. Selon un aspect supplémentaire, la combinaison selon l'invention comprend l'administration journalière de 1 à 100 mg de curcuminoïdes, de préférence de 5 à 50 mg de curcuminoides, par exemple de 10 à 30 mg ou de 10 à 20 mg de curcuminoïdes, lesdits curcuminoïdes comprenant au moins de la curcumine, et de préférence de la curcumine, de la déméthoxycurcumine et de la bisdéméthoxycurcumine, et lesdits curcuminoïdes étant administrés sous la forme d'un extrait de curcuma.

De préférence, l'extrait de curcuma est tel que décrit ci-avant. De préférence, l'extrait de curcuma est associé à une gamma-cyclodextrine et le rapport massique de curcuminoïdes sur la gamma-cyclodextrine est compris dans une gamme allant de 1 à 10, de préférence de 2 à 8 et de manière plus préférée de 3 à 7 par exemple de 4 à 5.

### 3.Régime d'administration de la combinaison selon l'invention

La combinaison ou association selon l'invention peut être administrée au sujet d'intérêt par toute voie adaptée, par exemple par voie buccale, orale, nasale, transmucosale par exemple vaginale, intrautérine, intrarectale, ou transdermale.

De préférence, la combinaison selon l'invention est administrée par voie orale.

Les ingrédients de la combinaison selon l'invention, à savoir le resvératrol, l'extrait de curcuma et la levure *Saccharomyces cerevisiae* peuvent être administrés simultanément au sujet. Par exemple, le resvératrol, l'extrait de curcuma et la levure *Saccharomyces cerevisiae* peuvent être présents dans la même composition pharmaceutique, nutraceutique ou alimentaire. Alternativement, le resvératrol, l'extrait de curcuma et la levure *Saccharomyces cerevisiae* peuvent être administrées de manière séparée, successivement ou séparée dans le temps. Par exemple, le resvératrol, l'extrait de curcuma et la levure *Saccharomyces cerevisiae* peuvent être administrés sous la forme d'un kit c'est-à-dire sous la forme de plusieurs compositions pharmaceutiques, nutraceutiques ou alimentaires distinctes, par exemple sous la forme d'aux moins deux unités de dose pour l'administration orale, par exemple d'une première gélule contenant la levure et d'un comprimé ou d'une deuxième gélule contenant le resvératrol et l'extrait de curcuma. Le kit d'administration de la combinaison peut alternativement comprendre trois gélules distinctes, une gélule contenant la levure, un comprimé ou gélule contenant le resvératrol, et un comprimé ou gélule contenant le l'extrait de curcuma.

Les doses, la fréquence d'administration et la durée du traitement est fonction des caractéristiques du sujet notamment de son âge, son poids, son historique médical, du stade et de la gravité de l'endométriose ou des symptômes ou troubles associés, des traitements médicaux qu'il prend etc...

Typiquement, la combinaison selon l'invention, peut être administrée au moins une fois par mois, par semaine ou par jour. De préférence, la combinaison selon l'invention est administrée quotidiennement pendant au moins une semaine, typiquement pendant au moins deux semaines, au moins trois semaines, au moins quatre semaines, au moins pendant 30 jours, voire pendant au moins 2, 3, 4, 6, 12 mois voire plus.

A titre d'exemple, la combinaison selon l'invention peut être administrée quotidiennement pendant 1 à 3 mois, ce traitement pouvant être renouvelé au besoin.

La combinaison selon l'invention est typiquement administrée en une dose efficace ou suffisante pour obtenir un des effets escomptés, tels que décrits ci-avant, notamment un effet permettant de diminuer, ralentir ou traiter un trouble ou symptôme associé à l'endométriose notamment en lien avec le développement des lésions d'endométriose et/ou permettant d'améliorer la condition générale d'un sujet souffrant d'endométriose.

Il est décrit dans une méthode de traitement de l'endométriose, qu'une "quantité thérapeutiquement efficace" d'une composition peut correspondre à une quantité qui réduit la sévérité d'un symptôme et/ou réduit un paramètre mesurable associé à la pathologie d'au moins environ 5%, 10%, au moins environ 20%, au moins environ 30%, au moins environ 40%, au moins environ 50%, au moins environ 60%, ou plus, en comparaison avec le symptôme (par exemple, la sévérité du symptôme), ou en comparaison avec le paramètre mesurable, en l'absence de traitement du sujet. Par exemple il peut s'agir d'un effet de ralentissement ou de diminution des lésions d'endométriose visualisable par exemple par imagerie (échographie, IRM et autres techniques de tomographie), d'un effet d'amélioration de la fertilité ou une diminution de l'inflammation mesurable, par exemple via certains marqueurs sanguins ou encore une diminution de l'intensité des douleurs mesurable à l'aide d'une échelle analogique de score de douleur.

Typiquement, les doses journalières à administrer peuvent aller :
- de 10 à 300 mg, de préférence de 25 à 250 mg de resvératrol,
- de 1 à 300 mg, par exemple de 10 à 300 mg, de 25 à 250 mg d'un extrait de curcuma, ou de préférence de 5 à 25 mg d'extrait de curcuma et
- de 1×10⁷ CFU à 1×10¹¹ CFU, de préférence de 1×10⁸ CFU à 1×10¹⁰ CFU ou de 1×10⁸ CFU à 1×10⁹ CFU ou de 5 à 100 mg, voire de 5 à 50 mg de levure *Saccharomyces cerevisiae,* de de variété boulardii.

A titre d'exemple supplémentaire, les doses journalières à administrer peuvent aller :
- de 30 mg à 70 mg, de préférence de 40 mg à 60 mg de resvératrol,
- de 5 à 100 mg, de préférence de 5 à 25 mg de curcuminoides sous la forme d'extrait de curcuma, et
- de 2×10⁸ CFU à 6×10⁸ CFU, de préférence de 3×10⁸ CFU à 5×10⁸ CFU de levure *Saccharomyces cerevisiae* de variété boulardii.

Ces doses journalières peuvent être administrées en une, deux, trois ou voire quatre prises par jour. De préférence, la combinaison selon l'invention est administrée en une ou deux prises par jour, de préférence une fois par jour.

La composition selon l'invention peut être administrée par voie orale avant le repas.

Dans un mode de réalisation additionnel, la combinaison selon l'invention est administrée en association avec un ou plusieurs médicaments conventionnels utilisés pour la prise en charge de l'endométriose et des symptômes associés. Ces médicaments englobent sans y être limités les contraceptifs, notamment les contraceptifs oraux de type pilule progestative ou pilule combinée, les anti-douleurs, notamment le paracétamol et l'aspirine, les AINS, notamment l'ibuprofène et le flurbiprofène, ou encore les traitements de stimulation ovarienne utilisés en procréation médicalement assistée (PMA), notamment dans le cadre de protocole de fécondation in vitro.

La combinaison selon l'invention peut être également utilisée dans la prise en charge de l'endométriose en combinaison avec une chirurgie d'exérèse des lésions d'endométriose. A titre d'exemple supplémentaire, la combinaison selon l'invention peut être également utilisée dans la prise en charge de l'endométriose en combinaison avec une cure thermale. Par « cure thermale » on entend ici l'utilisation d'eaux minérales naturelles des sources thermales et de leurs produits dérivés (par exemple gaz et boues thermales). A titre d'exemple, la cure thermale peut être suivie pendant au minimum trois semaines consécutives, par exemple dans le but de soulager les douleurs associées à l'endométriose, et comprend notamment l'application de soins tels que l'irrigation vaginale, les compresses d'eau mère, la douche d'eau thermale à pression modulable, et les séances de mobilisation en piscine d'eau thermale.

Plus généralement, la combinaison selon l'invention peut être utilisée en combinaison avec un traitement par médecine douce, par exemple en association avec de l'acupuncture, de l'ostéopathie, du yoga, de la kinésithérapie, de la sophrologie, de la naturopathie, de l'autohypnose, de la méditation et/ou de la relaxation.

Dans un mode de réalisation particulier, la combinaison selon l'invention est administrée à une femme souffrant d'endométriose et qui va subir un traitement choisi parmi une chirurgie d'exérèse des lésions d'endométriose et/ou un traitement de PMA. Typiquement, la combinaison selon l'invention peut être administrée quotidiennement au moins pendant une semaine (par exemple pendant au moins 2, 3, 4, 5, 6, 7, ou 8 semaines) avant le traitement. Cette administration peut être combinée avec l'administration d'un contraceptif oral, typiquement une pilule oestroprogestative par exemple à base de levonorgestrel et éthinyloestradiol, ou encore d'un agoniste de GnRH.

Dans un autre mode de réalisation, la combinaison selon l'invention est administrée à une femme souffrant d'endométriose et qui a subi un traitement chirurgical d'exérèse des lésions d'endométriose.

La combinaison selon l'invention peut être en outre administrée avec d'autres actifs ayant un effet physiologique ou nutritionnel, tels que les prébiotiques, les vitamines, minéraux oligo-éléments ; les poudres ou extraits de plantes, fruits, légumes, algues ou champignons ; et les probiotiques et plus généralement avec toute substance physiologique à but nutritionnel ou santé, de préférence telles que définies dans l'Arrêté du 26 septembre 2016 établissant la liste des substances à but nutritionnel ou physiologique autorisées dans les compléments alimentaires et les conditions de leur emploi en France. Des exemples de telles substances sont détaillés ci-après dans la partie composition.

Dans un mode de réalisation particulier, la combinaison selon l'invention est administrée conjointement avec un actif choisi dans le groupe constitué par les vitamines, notamment la vitamine C, la vitamine D, la vitamine B6, la vitamine B12, l'acide folique et/ou l'acide 5-methyltetrahydrofolique (les deux appartenant au groupe de la vitamine B9), les minéraux tel que le zinc, le cuivre, le magnésium, le calcium, les oligoéléments tel que le sélénium, ou le manganèse, un probiotique tel que les espèces Lactobacillus spp, par exemple Lactobacillus acidophilus, lactobacillus rhamnosus, lactobacillus plantarum, lactobacillus casei, lactobacillus paracasei ou encore les espèces Bifidobacterium spp, les prébiotiques tels que l'inuline, l'inositol ou les fructo/galacto-oligosaccharides, les gommes (guar, acacia...), les agents contraceptifs et leur combinaisons.

A titre d'exemple, la combinaison selon l'invention peut être administrée avec une ou plusieurs des substances suivantes : la vitamine B6, la vitamine B9, le zinc, le magnésium, le sélénium, l'inuline, un fructo ou galacto-oligosaccharide et leurs combinaisons.

A titre d'illustration, la combinaison selon l'invention est administrée en association avec du zinc (Zn2+), par exemple sous forme de sel de bisglycinate et d'un prébiotique comme l'inuline.

### 4.Composition et kit selon l'invention

L'invention a également pour objet une composition comprenant la combinaison d'actifs selon l'invention, à savoir le resvératrol, un extrait de curcuma et une levure *Saccharomyces cerevisiae.* Il va de soi que ladite composition est destinée notamment à être administrée aux sujets souffrant d'endométriose ou susceptibles de souffrir d'endométriose. Ladite composition est notamment adaptée pour la mise en oeuvre de l'une quelconque des utilisations et méthodes décrites dans la présente demande, notamment pour la prise en charge d'un sujet souffrant ou susceptible d'endométriose.

Le resvératrol, l'extrait de curcuma et la levure *Saccharomyces cerevisiae* sont tels que définis ci-avant.

La composition selon l'invention est caractérisée en ce que l'extrait de curcuma est un extrait comprenant au moins 15% en poids de curcuminoïdes et la levure *Saccharomyces cerevisiae* est une levure de la variété boulardii.

De préférence, la composition selon l'invention est une composition pour l'administration orale. Il peut s'agir, notamment, d'une composition pharmaceutique, nutraceutique ou alimentaire de préférence destinée à l'administration chez l'homme. Il peut s'agir notamment d'un médicament, d'un complément alimentaire, d'un aliment fonctionnel, d'un alicament, d'une préparation alimentaire destinée à un groupe spécifique...

De préférence, la composition selon l'invention est un médicament ou un complément alimentaire, de manière plus préférée un complément alimentaire.

La composition selon l'invention peut prendre de multiples formes. Il peut s'agir d'une composition solide, liquide ou de gel. De préférence, la composition selon l'invention est une composition sous une forme adaptée pour une administration par voie orale. Elle peut se présenter notamment sous la forme de pilules, comprimés, gélules poudres éventuellement à dissoudre ou à re-suspendre dans un véhicule adapté, de pâte ou gomme à mâcher, de bonbons à sucer ou à croquer, de solutions, par exemple solutions buvables conditionnées dans des ampoules, de gel, etc.

Dans certains modes de réalisation, la composition selon l'invention comprend, ou consiste essentiellement en, du resvératrol, un extrait de curcuma et des levures *Saccharomyces cerevisiae* selon les proportions suivantes :
- de 10 à 50 % en poids de resvératrol,
- de 10 à 60% en poids d'un extrait de curcuma,
- de 1 à 50% en poids de levures *Saccharomyces cerevisiae,*
les pourcentages étant exprimés pour 100% en poids de la somme de resvératrol, d'extrait de curcuma et de *Saccharomyces cerevisiae* présents dans ladite composition ou pour 100% en poids de la composition.

Dans un autre mode de réalisation, la composition selon l'invention comprend, ou consiste essentiellement en, du resvératrol, un extrait de curcuma et des levures *Saccharomyces cerevisiae* selon les proportions suivantes :
- de 20 à 70 % en poids de resvératrol,
- de 1 à 20% en poids d'un extrait de curcuma,
- de 10 à 60% en poids de levures *Saccharomyces cerevisiae,*
les pourcentages étant exprimés pour 100% en poids de la somme de resvératrol, d'extrait de curcuma et de levure *Saccharomyces cerevisiae* présents dans ladite composition ou pour 100% en poids de la composition. De préférence, les pourcentages sont exprimés pour 100% en poids de la somme de resvératrol, d'extrait de curcuma et de levure *Saccharomyces cerevisiae* présents dans ladite composition.

Dans un autre mode de réalisation, la composition selon l'invention comprend, ou consiste essentiellement en, du resvératrol, un extrait de curcuma et des levures *Saccharomyces cerevisiae* selon les proportions suivantes :
- de 20 à 40 % en poids de resvératrol,
- de 40 à 60% en poids d'un extrait de curcuma,
- de 5 à 30% en poids de levures *Saccharomyces cerevisiae,*
les pourcentages étant exprimés pour 100% en poids de la somme de resvératrol, d'extrait de curcuma et de levure *Saccharomyces cerevisiae* présents dans ladite composition ou pour 100% en poids de la composition. De préférence, les pourcentages sont exprimés pour 100% en poids de la somme de resvératrol, d'extrait de curcuma et de levure *Saccharomyces cerevisiae* présents dans ladite composition.

Dans un mode de réalisation supplémentaire, la composition selon l'invention comprend, ou consiste essentiellement en, du resvératrol, un extrait de curcuma et des levures *Saccharomyces cerevisiae* selon les proportions suivantes :
- de 50 à 70 % en poids de resvératrol,
- de 5 à 20% en poids d'un extrait de curcuma,
- de 15 à 40% en poids de levures *Saccharomyces cerevisiae,*
les pourcentages étant exprimés pour 100% en poids de la somme de resvératrol, d'extrait de curcuma et de levure *Saccharomyces cerevisiae* présents dans ladite composition ou pour 100% en poids de la composition. De préférence, les pourcentages sont exprimés pour 100% en poids de la somme de resvératrol, d'extrait de curcuma et de levure *Saccharomyces cerevisiae* présents dans ladite composition.

Dans un autre mode de réalisation, la composition selon l'invention comprend, ou consiste essentiellement en, du resvératrol, un extrait de curcuma et des levures *Saccharomyces cerevisiae* selon les proportions suivantes :
- de 25 à 35 %, e.g. de 30 à 34% en poids de resvératrol,
- de 45 à 55%, e.g. de 46 à 50% en poids d'un extrait de curcuma,
- de 15 à 25%, e.g. de 18 à 22% en poids de levures *Saccharomyces cerevisiae,*
les pourcentages étant exprimés pour 100% en poids de la somme de resvératrol, d'extrait de curcuma et de *Saccharomyces cerevisiae* présents dans ladite composition ou pour 100% en poids de la composition.

Dans certains modes de réalisation supplémentaires ou alternatifs, la composition selon l'invention est caractérisée par l'une ou plusieurs des caractéristiques suivantes :
- l'extrait de curcuma comprend de la curcumine, de la déméthoxycurcumine et/ou de la bisdéméthoxycurcumine, et/ou
- l'extrait de curcuma comprend au moins 55%, de préférence au moins 60% en poids de curcumine par rapport au poids total des curcuminoïdes présents dans l'extrait, et/ou
- l'extrait de curcuma est encapsulé dans une cyclodextrine, de préférence une gamma cyclodextrine, et/ou
- la composition comprend de 1% à 20%, de préférence de 1% à 10% en poids de curcuminoïdes pour 100% en poids de la somme de resvératrol, extrait de curcuma et *Saccharomyces cerevisiae* ou pour 100% en poids de la composition, et/ou
- la composition comprend de 0,5 à 10% en poids de curcumine pour 100% en poids de la somme de resvératrol, extrait de curcuma et *Saccharomyces cerevisiae* ou pour 100% en poids de la composition, et/ou
- le resvératrol est apporté sous la forme d'un extrait de plante enrichi en resvératrol, de préférence un extrait de renouée du Japon et/ou
- le resvératrol est apporté sous la forme de trans-resvératrol.

Il va de soi que la composition selon l'invention peut comprendre un ou plusieurs ingrédients supplémentaires par exemple un ou plusieurs additifs, excipients ou actifs supplémentaires tels que définis ci-après. Ce ou ces ingrédients supplémentaires sont de préférence choisis parmi les ingrédients acceptables sur le plan pharmaceutique, nutraceutique et/ou alimentaire. Ils peuvent représenter de 1% à 95%, de préférence de 1% à 79%, par exemple de 10% à 50%, ou encore de 20% à 60%, en poids par rapport au poids total de la composition selon l'invention.

Ainsi la composition selon l'invention peut comprendre :
- de 5% à 99%, de préférence de 21% à 99% en poids de resvératrol, de levure *Saccharomyces cerevisiae* et d'extrait de curcuma, de préférence selon les rapports ci-dessus mentionnés, et
- de 1% à 95%, de préférence de 1% à 79% en poids d'un ou plusieurs ingrédients choisis parmi les additifs, excipients et actifs supplémentaires acceptables sur le plan pharmaceutique, nutraceutique et/ou alimentaire.

A titre d'exemple supplémentaire, la composition selon l'invention peut comprendre :
- de 10 à 50 % en poids de resvératrol,
- de 10 à 60% en poids d'un extrait de curcuma,
- de 1 à 50% en poids de levures *Saccharomyces cerevisiae,* et
- de 1% à 79% poids d'un ou plusieurs ingrédients supplémentaires choisis parmi les additifs, excipients et actifs supplémentaires acceptables sur le plan pharmaceutique, nutraceutique et/ou alimentaire.

A titre d'autre exemple, la composition selon l'invention peut comprendre :
- de 10 à 50 % en poids de resvératrol,
- de 1 à 20% en poids de curcuminoides sous forme d'un extrait de curcuma,
- de 1 à 50% en poids de levures *Saccharomyces cerevisiae,* et
- de 1% à 88% poids d'un ou plusieurs ingrédients supplémentaires choisis parmi les additifs, excipients et actifs supplémentaires acceptables sur le plan pharmaceutique, nutraceutique et/ou alimentaire.

Les ingrédients supplémentaires peuvent être de tout type et sont fonction de la forme et des effets thérapeutiques, nutraceutiques, ou physiologiques recherchées pour la composition selon l'invention.

Dans un mode de réalisation particulier, la composition comprend un ou plusieurs actifs pharmaceutiques ou nutraceutiques additionnels, y compris une ou plusieurs substances physiologiques à but nutritionnel ou santé, de préférence telles que définies dans l'Arrêté du 26 septembre 2016 établissant la liste des substances à but nutritionnel ou physiologique autorisées dans les compléments alimentaires et les conditions de leur emploi en France. Le ou les plusieurs actifs pharmaceutiques ou nutraceutiques additionnels représentent, de préférence, au plus 50%, notamment au plus 45, 40, 30, 20, 15 ou 10% en poids par rapport au poids total de la composition. Typiquement, ils sont présents en une quantité allant de 0,5% à 30% en poids du poids final de la composition.

Les actifs pharmaceutiques englobent notamment les AINS, les contraceptifs notamment les progestatifs tels que le désogestrel et le lévonorgestrel et le diénogest les oestrogènes tels que l'éthinyloestradiol, les agonistes de GnRH et leurs combinaisons.

Les actifs nutraceutiques d'intérêt notamment à but nutritionnel ou physiologique englobent, sans y être limités, les vitamines, les minéraux, les oligoéléments, les extraits et poudres de plantes, algues ou champignons, les prébiotiques, les probiotiques, les acides aminés, les antioxydants et leurs combinaisons.

A titre d'exemple de vitamines, minéraux et oligo-éléments, on peut citer une vitamine (la thiamine (B 1), la riboflavine (B2), la niacine (B3), l'acide pantothénique (B5), la pyridoxine (B6), l'acide folique (B9) et la cyanocobalamine (B12), ainsi que les vitamines C, A, D, E, K1 et K2, un minéral tel que le magnésium, le calcium, ou le fer, des oligoéléments comme l'iode, le fer, le cuivre, le zinc, le sélénium, le chrome, le molybdène, le bore, le manganèse, ou un de leurs mélanges.

A titre d'exemple, les poudres ou extraits (secs ou liquides) de plantes, algues ou champignons peuvent notamment être choisis parmi les différentes listes réglementaires européennes connues de l'homme du métier comprenant par exemple: poudre/extrait de konjac, poudre/extrait d'ignames, poudre/extrait de haricots, poudre/extrait de café, poudre/extrait de thé, poudre/extrait d'écorce de pin, poudre/extrait de raisin, poudre/extrait d'ail, poudre/extrait de safran poudre/extrait de pomme, poudre/extrait de reishi, poudre/extrait de spiruline, poudre/extrait de chlorelles, poudre/ extrait d'algues brunes et tout mélange de ceux-ci.

A titre d'exemple, le prébiotique peut être un sucre, un alcool de sucre, un oligosaccharide ou un polysaccharide plus ou moins ramifié et de degré de polymérisation variable. Le prébiotique peut par exemple être choisi parmi l'inuline, l'inositol, le tagatose, le lactulose, l'alphaglucane oligosaccharide, les transgalacto-oligosaccharides (TOS), les fructo-oligosaccarides (FOS), les galacto-oligosaccharides (GOS), les xylo-oligosaccharides (XOS) et leurs mélanges.

A titre d'exemple, les acides aminés englobent, sans s'y limiter, la leucine, la citrulline, la glutamine, le glutamate, la cystéine et ses dérivés, la méthionine et ses dérivés, le tryptophane et ses dérivés.

A titre d'exemple, les antioxydants englobent, sans y être limités, la super oxyde dismutase (SOD), l'ubiquinol/ubiquinone (Coenzyme Q10), le resvératrol, les catéchines (catéchine, épicatéchine et dérivés gallatés), les flavanols, les flavanones ou encore les anthocyanes.

Les probiotiques englobent notamment les microorganismes choisis dans le groupe constitué par les microorganismes de genre *Bacillus, Bacteroides, Bifidobacterium, Enterobacteriaceae, Enterococcus, Faecalibacterium, Fusobacterium, Kluyveromyces, Lactobacillus, Odoribacter, Parabacteroides, Pediococcus, Ruminococcus, Streptococcus* ou autres levures.

Dans un mode de réalisation particulier, la composition selon l'invention comprend moins de 20 souches de microorganismes différentes, de préférence moins de 10, 9, 8, 7, 6, 5, 4, ou 3 souches de microorganismes différentes.

Par ailleurs ou en outre, la composition pharmaceutique, nutraceutique ou alimentaire peut également comprendre un ou plusieurs prébiotiques.

Selon un mode de réalisation, la composition pharmaceutique, nutraceutique ou alimentaire selon l'invention comprend un probiotique sous forme vivante ainsi qu'un ou plusieurs prébiotiques pouvant être dégradés par le probiotique ou le microbiote intestinal. Cette association de prébiotique(s) et probiotique constitue un symbiotique.

Dans un mode de réalisation particulier, la composition selon l'invention comprend une souche de *Lactobacillus* notamment *Lactobacillus rhamnosus* ou *acidophilus* comme probiotique additionnel.

Par exemple, la composition selon l'invention comprend une souche de Saccharomyces cerevisiae, de préférence de variété boulardii et une souche *de Lactobacillus,* de préférence *Lactobacillus acidophilus.*

Dans un autre mode de réalisation, la composition est dépourvue de tout probiotique appartenant au genre Lactobacillus, notamment de *Lactobacillus acidophilus,* et/ou au genre *Bifidobacterium.*

Dans un mode de réalisation supplémentaire, la levure *Saccharomyces cerevisiae* est le seul probiotique présent dans la composition selon l'invention.

Dans un mode de réalisation particulier, la composition selon l'invention comprend un actif nutraceutique ou pharmaceutique additionnel choisi dans le groupe constitué par les vitamines, notamment la vitamine C, la vitamine B6, la vitamine B12, l'acide folique et/ou l'acide 5-methyltetrahydrofolique (vitamine B9), un oligoélément ou minéral tel que le zinc, le magnésium, le calcium, le sélénium, le manganèse, un probiotique tel que les espèces Lactobacillus spp, une substance à but nutritionnel ou physiologique tel que l'inuline, les fructo/galacto-oligosaccharides, les gommes (guar, acacia...) ou l'inositol et leur combinaison Dans un autre mode particulier de réalisation, la composition selon l'invention comprend un actif nutraceutique ou pharmaceutique additionnel choisi dans le groupe constitué par les vitamines, notamment la vitamine C, la vitamine D, la vitamine B6, la vitamine B12, l'acide folique et/ou l'acide 5-methyltetrahydrofolique, et un oligoélément ou minéral tel que le zinc, le magnésium, le calcium, le sélénium ou le manganèse.

A titre d'exemple, la composition comprend, en plus de la combinaison selon l'invention, une ou plusieurs des substances choisies dans le groupe constitué par la vitamine B6, la vitamine B9, le zinc, le magnésium, le sélénium, l'inuline, un fructo/galacto-oligosaccharide et leurs combinaisons.

Par exemple, la composition selon l'invention comprend du zinc et de la vitamine B6.

Selon un autre exemple, la composition selon l'invention peut comprendre du zinc et de l'inuline.

Dans un mode de réalisation particulier, la composition selon l'invention se présente sous la forme d'unités de dose comprenant :
- de 10 à 300 mg, de préférence de 25 à 250 mg de resvératrol,
- de 10 à 300 mg, de préférence de 25 à 250 mg d'un extrait de curcuma, et
- de 1×10⁷ CFU à 1×10¹¹ CFU, de préférence 1×10⁸ CFU à 1×10⁹ CFU de levure *Saccharomyces cerevisiae,* de préférence de variété boulardii.

Dans un autre mode de réalisation particulier, la composition selon l'invention se présente sous la forme d'unités de dose comprenant :
- de 25 à 100 mg, de préférence de 30 à 70 mg de resvératrol,
- de 1 à 300 mg, de préférence de 5 à 25 mg d'un extrait de curcuma, et
- de 1×10⁸ CFU à 1×10⁹ CFU, de préférence de 2×10⁸ CFU à 6×10⁸ CFU de levure *Saccharomyces cerevisiae,* de préférence de variété boulardii.

Les unités de dose peuvent être par exemple des comprimés et des gélules.

La formulation d'une composition pharmaceutique, nutraceutique ou alimentaire selon la présente invention peut varier en fonction de la voie d'administration et du dosage pour lesquels la composition est destinée à être utilisée. La composition pharmaceutique, nutraceutique ou alimentaire selon l'invention peut notamment être sous forme solide, semi-solide ou liquide. Après formulation avec au moins un véhicule ou excipient physiologiquement acceptable, une composition selon l'invention peut être sous toute forme appropriée pour l'administration à un mammifère, en particulier l'homme, par exemple sous la forme de comprimés, pastilles, dragées, capsules, gélules, pilules, granulats, poudre, suspensions, émulsions, sirops, onguents, ampoule de liquide, flacon muni d'un compte-goutte et autres formes analogues de préparations liquides ou en poudres, ou suppositoires.

L'homme de l'art sait sélectionner les véhicules et excipients les plus appropriés à la préparation d'un type donné de formulation.

Dans un mode de réalisation particulier, le véhicule physiologiquement acceptable peut être un solide et la composition selon l'invention peut être sous la forme d'une poudre ou d'un comprimé. Le véhicule physiologiquement acceptable peut alternativement être un liquide, et la composition selon l'invention est sous la forme d'une solution. Les véhicules liquides sont utilisés dans la préparation de solutions, de solvants, de milieux de dispersion, de suspensions, d'émulsions, de sirops, d'élixirs et de compositions sous pression. Les supports liquides ou à base de gel appropriés comprennent, sans s'y limiter : l'eau et les solutions salines physiologiques ; les émulsions ou les suspensions, y compris les solutions salines et les milieux tamponnés, l'urée ; les solvants non aqueux comme des alcools (par exemple, l'éthanol) ou les huiles végétales ou de graines telles que l'huile d'olive. Les compositions liquides, y compris par exemple les émulsions, microémulsions, solutions, suspensions, sirops, ou élixirs peuvent être formulés notamment en présence de solvants, d'agents solubilisants, d'émulsifiants, d'huiles, d'acides gras, et/ou d'autres additifs comme par exemple des agents de suspension, des conservateurs, des édulcorants, des arômes naturels ou synthétiques, des agents stabilisants et/ou épaississants et/ou des agents colorants de qualité alimentaire. Les émulsions peuvent contenir des agents émulsifiants tels que la lécithine, le monooléate de sorbitan ou l'acacia. Des agents épaississants bien connus peuvent également être ajoutés aux compositions telles que l'amidon de maïs, des gommes naturelles ou synthétiques, des résines, la méthylcellulose, la carboxyméthylcellulose sodique, la gomme de guar, la gomme de xanthane et analogues.

Les excipients et véhicules sont choisis de manière à ne pas affecter de manière significative la viabilité de la levure *Saccharomyces cerevisiae* et autres probiotiques éventuellement présents dans la composition selon l'invention.

En particulier, la composition selon l'invention peut comprendre en outre :
- des anti-agglomérants comme le carbonate de magnésium, le carbonate de calcium, le dioxyde de silicium, l'acide stéarique, un sel d'acide gras par exemple du stéarate de magnésium, du palmitate de magnésium, de l'oléate de magnésium ou du myristate de magnésium ; et/ou
- un ou plusieurs agents de viscosité ou tensio-actifs, notamment l'amidon, les éthers de cellulose comme l'hydroxypropylméthylcellulose ou la gomme arabique ; et/ou
- un ou plusieurs agents liants, notamment l'huile de poisson ou toute huile végétale telle que par exemple l'huile de tournesol, l'huile de soja, l'huile d'olive ou l'huile de chia ; et/ou
- des agents d'enrobage comme la cire d'abeille, la cire de canauba ou les monoglycérides d'acides gras alimentaires ; et/ou
- des émulsifiants comme la lécithine, les cyclodextrines ou la gomme ester ; et/ou
- un ou plusieurs diluants comme la cellulose ou la maltodextrine
- un ou plusieurs agents édulcorants non nutritifs, notamment le sorbitol, le sucralose, l'aspartame, le néotame, le maltitol, le xylitol, l'acésulfame-K, l'aspartame, la saccharin, l'acide glycyrrhizique ou un extrait de stévia ; et/ou
- un ou plusieurs agents de saveur, naturels ou artificiels, sous forme pulvérulente ou liquide ; et/ou
- un ou plusieurs correcteurs d'acidité, notamment le sel, l'acide citrique, l'acide malique ou l'acide tartrique ; et/ou
- un ou plusieurs colorants, notamment les anthocyanes, le béta carotène, l'extrait de betterave, le lycopène ou l'extrait de caramel.

Dans certains modes de réalisation, une composition pharmaceutique, nutraceutique ou alimentaire selon la présente invention est formulée pour une libération immédiate du ou des principes actifs. Alternativement, une composition pharmaceutique peut être formulée pour une libération prolongée ou ciblée du ou des principes actifs ou pour une protection du ou des principes actifs, par exemple vis-à-vis de l'acidité et des enzymes gastriques. Il est ainsi possible d'utiliser des enrobages résistants au pH et/ou à l'action des enzymes gastriques, des enrobages sensibles au pH et/ou à des actions enzymatiques, ou des enrobages bioadhésifs qui s'accrochent aux parois de l'estomac ou de l'intestin, ou des systèmes d'encapsulation.

De préférence, la composition est sous une forme orale gastro-résistante permettant aux levures contenues dans la composition selon l'invention de résister au suc gastrique de l'estomac et d'être libérées dans l'intestin. Un enrobage entérique peut être stable à pH acide (comme dans l'estomac) et peut se dissoudre à un pH basique (par exemple, dans l'intestin). Le matériau qui peut être utilisé dans les enrobages entériques comprend, par exemple, l'acide alginique, le phtalate d'acétate de cellulose, les cires, la gomme laque, les acides gras (par exemple l'acide stéarique ou l'acide palmitique) ou encore le chitosan de façon non exclusive.

Dans un mode de réalisation, le revêtement gastro-résistant et entéro-soluble est conçu pour maintenir la stabilité des ingrédients actifs dans l'estomac. Le revêtement entérique est conçu pour se maintenir dans des conditions acides de l'estomac et se dégrader dans des conditions non acides, libérant ainsi la composition pharmaceutique, nutraceutique ou alimentaire dans les intestins.

Les enrobages entériques peuvent être utilisés pour i) empêcher le suc gastrique de réagir avec ou détruire la substance active, ii) empêcher la dilution de la substance active avant qu'elle n'atteigne l'intestin, iii) s'assurer que la substance active n'est libérée qu'après le passage de la préparation à travers l'estomac, et iv) empêcher les levures vivantes contenues dans la composition selon l'invention d'être altérées ou tuées par le pH acide de l'estomac.

Selon un mode de réalisation, la composition peut comprendre en outre de la maltodextrine et/ou du stéarate de magnésium.

La composition selon l'invention peut être préparée par toute méthode connue par l'homme du métier par exemple en mélangeant le resvératrol, l'extrait de curcuma et la levure *Saccharomyces cerevisiae* avec un ou plusieurs excipients ou additifs acceptables sur le plan alimentaire, nutraceutique ou pharmaceutique.

Dans un mode de réalisation particulier, la composition selon l'invention se présente sous la forme d'unités de dose comprenant :
- de 25 à 100 mg, de préférence de 30 à 70 mg de resvératrol,
- de 1 à 300 mg, de préférence de 5 à 25 mg d'un extrait de curcuma, et
- de 1×10⁸ CFU à 1×10⁹ CFU, de préférence de 2×10⁸ CFU à 6×10⁸ CFU de levure *Saccharomyces cerevisiae* de variété boulardii,
- un prébiotique, de préférence l'inuline, et
- du zinc, de préférence sous forme de bisglycinate de zinc.

La composition selon l'invention peut en outre comprendre un ou plusieurs excipients choisis parmi le groupe constitué par un diluant, un anti-agglomérant, un émulsifiant, un agent de viscosité et leurs combinaisons.

En particulier, la composition selon l'invention peut comprendre de la gamma-cyclodextrine, de la maltodextrine et un sel d'acides gras, par exemple du stéarate de magnésium ou un mélange de sels de magnésium d'acides stéarique, oléique, palmitique et myristique

De préférence, les unités de dose selon l'invention se présentent sous la forme d'une poudre. Cette poudre est typiquement contenue dans une gélule, de préférence une gélule à base d'hypromellose (HPMC) et de carbonate de calcium.

A titre d'illustration, la composition selon l'invention se présente sous la forme d'unités de dose comprenant :
- de 25 à 100 mg, de préférence de 30 mg à 70 mg de resvératrol, de préférence trans-resvératrol,
- de 5 à 25 mg de curcuminoides sous la forme d'un extrait de curcuma,
- de 1×10⁸ CFU à 1×10¹⁰ CFU, de préférence de 2×10⁸ CFU à 6×10⁸ CFU, de manière plus préférée de 3×10⁸ CFU à 5×10⁸ CFU de levure *Saccharomyces cerevisiae* de variété boulardii, par exemple la souche CNCM I-3799 ou la souche CNCM I-745
- de 10 mg à 100 mg, de préférence de 25 mg à 50 mg d'inuline, par exemple fournie sous la forme d'un extrait de racine de chicorée
- de 1 mg à 15 mg de zinc (Zn2+) de préférence de 2 mg à 8 mg de zinc, par exemple sous forme de bisglycinate de zinc.

L'unité de dose peut être contenue dans une gélule de préférence végétale à base d'HPMC ou de pullulane.

L'unité de dose peut comprendre, en tant qu'excipients, de la gamma-cyclodextrine, de la maltodextrine et un sel de magnésium d'acides gras, par exemple un sel de magnésium d'acide stéarique, oléique, palmitique, myristique et leur mélange.

La gamma-cyclodextrine est typiquement présente pour améliorer la biodisponibilité de l'extrait de curcuma. La gamma-cyclodextrine peut être présente en une quantité allant de 20 à 120 mg par unité de dose. La maltodextrine peut être présente en une quantité allant de 5 à 50 mg par unité de dose. Le sel de magnésium d'acide gras peut être présent en une quantité allant de 0,1 mg à 10 mg par unité de dose.

Un objet supplémentaire selon l'invention est un kit nutraceutique ou pharmaceutique, par exemple un kit de complément alimentaire comprenant plusieurs compositions, de préférence destinées à l'administration orale, intégrant la combinaison selon l'invention.

Dans un mode de réalisation particulier, ledit kit comprend :
- une composition comprenant la levure *Saccharomyces cerevisiae,*
- une composition comprenant le resvératrol, et
- une composition comprenant l'extrait de curcuma.

Dans un mode particulier de réalisation, le kit comprend :
- une première composition comprenant la levure *Saccharomyces cerevisiae,* par exemple associée à un ou plusieurs excipients dans une gélule,
- une seconde composition comprenant le resvératrol, et l'extrait de curcuma, par exemple sous la forme d'un comprimé ou d'une gélule.

Le kit comprend typiquement plusieurs unités de dose de chaque composition. Il peut comprendre également des instructions d'administration du kit.

Le kit selon l'invention est caractérisé en ce que l'extrait de curcuma est un extrait comprenant au moins 15% en poids de curcuminoïdes et la levure *Saccharomyces cerevisiae* est une levure de la variété boulardii.

Dans un mode de réalisation particulier, la première composition peut se présenter sous la forme d'unités de dose comprenant de 10 à 300 mg, de préférence de 25 à 250 mg de resvératrol et de là 300 mg, de préférence de 10 à 300 mg, de 5 à 25 mg ou de 25 à 250 mg d'un extrait de curcuma, et la seconde composition peut se présenter sous la forme d'unités de dose comprenant de 1×10⁷ CFU à 1×10¹¹ CFU, de préférence 1×10⁸ CFU à 1×10⁹ CFU de levure *Saccharomyces cerevisiae,* de variété boulardii. Les unités de dose peuvent être par exemple des comprimés et des gélules.

Les caractéristiques des compositions présentes dans ce kit sont similaires à celles de la composition selon l'invention notamment, en termes de rapport massique de levure, resvératrol et extrait de curcuma entre eux, et de doses.

Chaque composition présente dans le kit peut comprendre un ou plusieurs ingrédients supplémentaires choisis parmi les additifs, excipients et actifs supplémentaires acceptables sur le plan pharmaceutique, nutraceutique et/ou alimentaire tels que décrits ci-avant.

L'invention sera mieux comprise à la lecture des exemples qui suivent, qui se veulent uniquement illustratifs et non limitatifs.

### EXEMPLES

### Exemple 1 : Evaluation de l'effet de la combinaison selon l'invention selon l'invention sur un modèle murin d'endométriose

L'objectif de cette étude était de tester *in vivo* l'effet d'une combinaison selon l'invention comprenant un extrait de curcuma, du resvératrol et une levure *S. cerevisae* var. *boulardii* sur la croissance de lésions d'endométriose et le statut immunitaire et inflammatoire chez un modèle murin d'endométriose.

### Matériel et méthodes

Les souris Balb/CJrj femelles agées de 6 semaines ont été achetées chez le fournisseur Janvier Labs (Route du Genest, 53940 Le Genest-Saint-Isle). Les souris sont hébergées dans une animalerie conventionnelle, avec des cycles jour/nuit automatisés de 12h, boisson (eau du robinet) et nourriture (Teklad, Envigo, Gannat, France, #T.2016MI.12) *ad libitum.* Le modèle de souris endometriosiques par implantation (suture) de corne utérine syngénique intra-péritonéale est un modèle validé et plusieurs fois utilisé et publié [Jones *et al.*, 2018 ; Ruiz *et al.*, 2016]. L'implantation de cornes utérines en intra- péritonéale est effectuée sous anesthésie à l'isoflurane (3,5 %, sous oxygène 2,5 l/min) et d'analgésique en post-opératoire (Buprécare, 10µg/kg, sc). Une laparotomie est réalisée et un fragment est fixé de chaque côté de l'ouverture, sur la paroi interne du péritoine, par deux points de suture réalisé avec du fil chirurgical résorbable 5/0.

Les molécules et produits utilisées pour les traitements sont : Zinc-Bisglycinate Chelate (ALBION Laboratories, Clearfield, USA, #03506), Gomme Acacia Seygal (Nexira Food, Rouen, France, #FibregumB), Trans-resvératrol (Active Inside, Beychac et Caillau, France, #PR-0018), extrait de curcuma (Wacker, Eddyville,IA, USA, #60072151), Saccharomyces Boulardii (LHC Lesaffre Bio Springer, Maison Alfort, France, #VI1700368), Lactobacillus Acidophilus (Danisco US, Madison, WI, USA, #MSAMPHRUDOPH).

Les groupes de souris sont les suivants :
- Groupe Contrôle (Sham) (n=10) : Souris pseudo-opérées mais sans implant et sans traitement (Sham) ;
- Groupe M (contrôle) (n= 10) : souris opérées, traitées avec une composition comprenant le Véhicule (Zinc-Bisglycinate, Gomme Acacia-Seygal, Trans-resvératrol et Extrait de curcuma) et donc sans probiotique;
- Groupe M1 (Invention) (n= 20): souris opérées, traitées avec une composition comprenant le Véhicule et 0,36 mg/j de Saccharomyces Boulardii ;
- Groupe M2 (Invention) (n= 20) : souris opérées, traitées avec une composition comprenant le Véhicule, 0,36 mg/j de Saccharomyces Boulardii et 0,18 mg/j de Lactobacillus Acidophilus) ; Les compositions ont été mises en suspension en phase aqueuse et ont été administrées *per os* par intubation gastrique à des souris (1 gavage par jour). La préparation des solutions est réalisée extemporanément et l'administration est effectuée le matin. Pour chaque groupe, l'administration des différentes solutions dure 12 semaines consécutives. A S3 (trois semaines) et S12 (12 semaines), les animaux seront sacrifiés (n= 5 par groupe Sham et M et n= 10 par groupe M1 et M2 à S3 et S12). Le sang, la rate et les lésions seront prélevés. La taille et le poids des lésions seront évalués et les analyses suivantes seront réalisées.

Les tableaux ci-dessous reprennent les caractéristiques de l'essai :

**Tableau 1. Répartition des animaux (n=10 par groupe)**

| Groupes | Traitements |
|---|---|
| Sham - Pseudo-opérées | / |
| Modèle induit + M | Resvératrol + Extrait de curcuma |
| Modèle induit + M1 | Resvératrol + Extrait de curcuma + Saccharomyces boulardii |
| Modèle induit + M2 | Resvératrol + Extrait de curcuma + Saccharomyces boulardii + *Lactobacillus acidophilus* |

**Tableau 2. Quantité de matières premières administrées par intubation gastrique aux souris**

| **Ingrédients** | **Quantités apportées/j (mg)** | **Quantités apportées par intubation gastrique (mg)** |
|---|---|---|
| Saccharomyces boulardii - LYNSIDE PRO^{®} - CNCM N° 1-3799 (groupe M1 et M2) | 0,36 | 0,36 |
| Lactobacillus acidophilus NCFM (groupe M2) | 0,18 | 0,18 |
| Extrait de polygonum à 98% Resveratrol | 0,6 | 0,6 |
| Extrait de curcuma | 0,9 | 0,9 |

### - Le suivi de l'évolution du volume des lésions est fait par échographie.

L'échographie haute résolution (40m) permet de suivre l'évolution de la taille des implants de cornes utérines de manière précise, non invasive et d'évaluer rapidement la progression de l'endométriose. Cette procédure est réalisée à la PIPA (Plateforme d'Imagerie du Petit Animal de l'Institut Cochin) à S1, S3, S8, S11 après l'implantation des fragments de cornes.

Après euthanasie des prélèvements sont réalisés (à S3 et S12) afin de mesurer divers paramètres.

### - Histologie des lésions par coloration Hemalun/Eosine (HE)

Les coupes de 5µm des lésions fixées en paraffine ont été colorées par la technique conventionnelle HE.

### - Statut immunitaire dans les rates par FACS (LT, NK, macrophages)

Des anticorps spécifiques ont été utilisés afin de déterminer les proportions de lymphocytes T CD4 et CD8, Natural Killer et macrophages spléniques :
MIX 1 : B220 APC, CD69 PE, CD8 BV605, CD40 PerCP/Cy5.5, MHCII FITC, CD4 BV510 - MIX 2 : CD206 AF647, CD86 BV510, Ly6C PE VIOL.770, CD11b APC-Cy7 et F4/86 BV711. Le FACS utilisé est un LSR Fortessa BD et le logiciel d'analyse BD FACS Diva 6.2 software.

### - Dosages dans les sera :

AOPP mesurées par spectrophotométrie sur le lecteur de plaques dans une plaque 96 puits d'après la techniques publiée (Kidney Int. 1996 May;49(5):1304-13. Advanced oxidation protein products as a novel marker of oxidative stress in uremia. Witko-Sarsat V et Al.) L'IL6 et le TNFa sont mesurés par kits ELISA (Thermofisher Scientific, Waltham, MA USA #KMC0061 et BMS607-3 respectivement) comme la Zonuline (Clinisciences, Nanterre, France, #MBS748504-96).

### - Etude des lésions par histologie et qRT-PCR

L'ARN total a été extrait des tissus de souris (lésions de type endométriose) en utilisant le réactif Trizol (Invitrogen, Carlsbad, USA), selon les instructions du fabricant. L'ADNc a été synthétisé en utilisant le kit de synthèse Maxima H Minus cDNA Synthesis Master Mix kit (Thermo Fisher Scientific). La qPCR a été réalisée en utilisant le kit SensiFAST SYBR No-ROX (Bioline). Les niveaux de transcription des gènes sélectionnés ont été quantifiés dans un Light Cycler 480 (Roche, San Francisco, CA) et normalisés pour les gènes de référence (Actinb et Tbp) et analysés grâce au logiciel LightCycler dédié.

### Etude statistique des différents résultats

L'analyse statistique comparative des différents groupes de souris est effectuée par ANOVA à 1 facteur suivie d'un test de Kruskal-Wallis. La comparaison S3-S12 par ANOVA suivie d'un test de Bonferroni.

### Résultats

### 1) Poids et volume des lésions endométriosiques

Les poids et volumes des lésions sont homogènes à l'implantation pour les trois groupes de souris M, M1 et M2 (M, n=10 ; M1, n=20 ; M2, n=20).

Après 12 semaines de traitement, on note que le volume et le poids des lésions sont significativement plus faibles chez les souris ayant été traitées avec une composition selon l'invention (M1 ou M2) que chez les souris traitées avec la composition contrôle M (p<000,1). Ainsi, la combinaison selon l'invention associant du resvératrol, un extrait de curcuma et la levure *Saccaromyces cerevisiae boulardii* agit de manière synergique sur le développement des lésions d'endométriose. (Figure 1).

### 2) Histologie des lésions prélevées au sacrifice

Histologiquement, on observe une diminution de l'activité des lésions dans les groupes M1 et M2 par rapport au contrôle M, après 3 et 12 semaines de traitement (Figure 2) : en effet, l'aspect glandulaire, un marqueur des lésions d'endométriose, est nettement atténué dans les groupes M1 et M2 par rapport au groupe M.

### 3) Statut immunitaire spléniques dans les différents groupes, à S12

Le statut immunitaire est modifié dans tous les groupes comparés au groupe pseudo-opéré sans implantation de lésion (SHAM) (Figures 3 et 4).

Les pourcentages de TCD4 et TCD8 activés de la rate tendent à diminuer sans significativité entre les groupes Sham, M, M1 et le groupe M2 (Sham, n=3 -M, n=6 - M1, n=5 - M2, n=5). Le pourcentage de cellules B activées de la rate, tend à être diminué par les formulations M, M1 et M2 (Sham, n=3 - M, n=6 - M1, n=5 - M2, n=5).

On note que le rapport M1/M2 (macrophages de type 1 et 2) est significativement augmenté chez les souris des groupes selon l'invention (M1 et M2) par rapport aux groupes contrôle (M et Sham). Or, des études chez l'homme et la souris ont mis en évidence le rôle clé des macrophages M2 dans la pathogenèse de l'établissement et de la croissance des lésions d'endométriose. En effet, il a été montré une élévation de la proportion de macrophages M2 dans le liquide péritonéal des femmes atteintes d'endométriose, par rapport aux témoins. De plus, il a été également montré que le transfert intrapéritonéal de macrophages M2 entraine la croissance et la néovascularisation d'endométriose chez la souris (Bacci et al., 2009). Ainsi, les résultats de la présente étude suggèrent que la combinaison selon l'invention a un effet immunomodulateur sur la polarisation des macrophages, ce qui pourrait permettre de normaliser la réponse immunitaire chez les patientes souffrant d'endométriose

### 4) Paramètres inflammatoires

La Zonuline, marqueur de perméabilité de la barrière intestinale, et l'Interleukine 6, marqueur d'inflammation, sont très significativement diminuées dans les groupes selon l'invention (M1 et M2, respectivement), comparé au groupe contrôle pseudo-opéré (SHAM). Le TNF-a est aussi diminué de façon très significative car non détectable dans les sérums des groupes M1 et M2 (Figure 5).

### 5) Stress oxydant

Les produits avancés protéiques d'oxydation (AOPP), marqueur du taux d'oxydation des protéines sériques et du stress oxydant, sont diminués significativement dans les trois groupes supplémentés (M, M1 et M2) à 12 semaines par rapport au groupe pseudo-opéré (Sham) (Figure 4).

### 6) Résultats qPCR

Les résultats préliminaires montrent une tendance à la diminution du marqueur d'angiogenèse (Cd31) pour les groupes M1 et M2 à S12 La comparaison S12-S3 par test ANOVA suivie d'un test de Bonferroni montre une diminution significative uniquement pour le marqueur Igf1 (marqueur de la douleur) pour le groupe M1.

Concernant la sensibilité à la douleur, il est à noter que la sensibilité à la chaleur était significativement diminuée dans le groupe M1 par rapport aux groupes M0 et M2 à la semaine 4 de traitement selon le test « hot plate ».

### En conclusion

Les résultats ci-avant montrent que la combinaison selon l'invention a des effets favorables aussi bien sur le plan clinique qu'immunitaire et physiologique. La combinaison selon l'invention ralentit de manière significative la croissance des lésions d'endométriose chez le modèle murin. Par ailleurs, l'endométriose est caractérisée par une fonction immunitaire perturbée. Or, la combinaison selon l'invention exerce des effets immunomodulateurs permettant de réduire l'inflammation comme le reflètent une baisse des taux sériques des cytokines pro-inflammatoires TNF-α et d'IL-6, la modulation du rapport des macrophages M1 / M2 et la réduction de l'activation des cellules B. Enfin la combinaison selon l'invention permet de réduire le taux circulant de zonuline, un marqueur de la perméabilité intestinale.

### Exemple 2 : Etude prospective, randomisée, en double aveugle, afin d'évaluer l'impact d'un complément alimentaire contenant la combinaison selon l'inventionsur la qualité de vie des femmes souffrant d'endométriose douloureuse

### • Centres investigateurs :

Hôpital COCHIN - Port Royal, France
**Méthodologie :**
Cette étude pilote est une étude monocentrique, randomisée, en bras parallèle, en double aveugle, contrôlée avec un placebo.

### • Produits:

- le produit expérimental (appelé ci-après Qolendo) est un complément alimentaire comprenant la combinaison selon l'invention (unité de dose : resvératrol 50 mg, S. boulardi : 400 millions d'UFC et curcumine 12,7 mg) de l'inuline (34 mg), du bisglycinate de zinc ( 20 mg eq., gamma-cyclodextrine (58,5 mg), stéarate de magnésium (1,5 mg) et maltodextrine (19,5 mg), sous forme de gélule (HPMC, carbonate de calcium).
- le produit de contrôle est une gélule placebo avec les mêmes caractéristiques d'emballage et d'aspect dans lequel les ingrédients actifs ont été remplacés par de la maltodextrine, du β-carotène (pour apporter une couleur orangée) et du stéarate de magnésium.

### • Population étudiée :

80 femmes souffrant d'endométriose douloureuse et correspondant aux critères d'inclusion et d'exclusion seront randomisées dans cette étude.

### • Planning de l'étude :

Recrutement : 10 mois, Durée de la prise de produit : 4 mois, Durée totale de participation par sujet : 5 mois, Durée totale de l'étude : 15 mois

### • Intervention :

Après la randomisation la visite V1, les sujets consommeront :
- le produit expérimental : groupe Qolendo
   1 gélule par jour de produit Qolendo de la visite 1 à la visite 3 (4 mois de prise de produit) le matin ou le soir, avec un verre d'eau.
   Ou
- le groupe contrôle : placebo
   1 gélule par jour de placebo de la visite 1 à la visite 3 (4 mois de prise de produit) le matin ou le soir, avec un verre d'eau.

### • Objectifs de l'étude

Hypothèse de l'étude: L'hypothèse de l'étude est que le produit testé Qolendo améliore la qualité de vie des femmes atteintes d'endométriose douloureuse.

**Objectif principal:** L'objectif principal est d'évaluer la qualité de vie chez les femmes atteintes d'endométriose du groupe Qolendo par rapport à celles du groupe placebo, à l'aide du questionnaire EHP-5 après 4 mois de prise de produit.

**Objectifs secondaires :** Les objectifs secondaires de l'étude sont d'évaluer les paramètres suivants, entre les 2 groupes et la différence intra-groupe :
1) La qualité de vie à l'aide du questionnaire EHP-5 après 2 mois de prise de produit,
2) Le questionnaire Clinical Global Impression Scale (CGI) après 4 mois de prise de produit,
3) Les douleurs ressenties pendant toute la durée de l'étude,
4) La consommation d'antalgiques pendant toute la durée de l'étude,
5) Le statut inflammatoire, immunitaire et hormonal après 4 mois de prise de produit,
6) La perméabilité de l'épithélium intestinal après 4 mois de prise de produit,
7) L'acceptabilité du produit dans le groupe Qolendo après 2 et 4 mois de prise du produit.

**Objectifs exploratoires:** L'échographie pelvienne réalisée dans un sous-groupe de femmes atteintes d'endométriose profonde et/ou avec présence d'adénomyose, Evaluation de la qualité de vie à l'aide du questionnaire EHP-30, complété sur la base du volontariat : ce questionnaire sera rempli à la visite de randomisation (V1) et après 4 mois de prise de produit lors de la visite V3.

**Objectifs de sécurité :** Les objectifs de sécurité de l'étude sont d'évaluer l'incidence des événements indésirables (EI) et l'état de santé général par le biais des signes vitaux, le poids corporel et paramètres biologiques (mesurés à 4 mois).

### • Critères de jugement

**Critère de jugement principal:** Le critère principal de cette étude est l'évolution de la qualité de vie, à 4 mois par rapport à la visite initiale (V1), évaluée par le questionnaire EHP-5, score global, exprimée en unité arbitraire (u.a., score), en comparant le groupe Qolendo versus le groupe placebo.

**Critères de jugement secondaire :** Les critères secondaires de cette étude listés ci-dessous permettent de comparer le groupe Qolendo et le groupe placebo :
1) L'évolution de la qualité de vie à 2 mois par rapport à la visite initiale, évaluée par le questionnaire EHP-5, exprimée en u.a.,
2) L'évolution du questionnaire Clinical Global Impression Scale à 4 mois par rapport à la visite initiale. Ce questionnaire est composé de 3 sous- échelles mesurant :
   a. La sévérité de la maladie (score variant de 1 normal à 7 cas les plus sévères),
   b. L'amélioration globale ou le changement (score variant de 1 forte amélioration à 7 forte dégradation),
   c. La réponse thérapeutique (score variant de 0 amélioration marquée sans évènements indésirables à 4 pas de changement ou détérioration et balance bénéfice/risque insatisfaisante).
3) L'évolution de la douleur, pendant toute la durée de l'étude par rapport à la valeur initiale, définie sur des échelles visuelles analogiques (EVA) graduée de 0 à 10 évaluant :
   Douleur (évaluation globale), Dysménorrhée, Dyspareunie, Douleur pelvienne chronique non cyclique, Douleur digestive, Dyschésie, Douleur mictionnelle. Les dates de menstruation seront collectées afin de distinguer les douleurs cataméniales.
4) La consommation quantitative d'antalgique (en g/jour) pendant toute la durée de l'étude, par rapport à la valeur initiale,
5) Le statut inflammatoire à 4 mois comparativement à la valeur initiale évalué par les paramètres suivants : TNF-a, IL1, IL6, CRP-us, AOPP,
6) Le statut immunitaire à 4 mois comparativement à la valeur initiale évalué par les paramètres suivants : IL4, IL10, IL12, INFγ, cellules NK ,
7) Le statut hormonal à 4 mois comparativement à la valeur initiale évalué par l'estradiol (E2), la progestérone et l'hormone anti-müllerienne (AMH),
8) La perméabilité de l'épithélium intestinal à 4 mois comparativement à la valeur initiale évaluée par le dosage sanguin de la zonuline,
9) L'acceptabilité du produit dans le groupe Qolendo après 2 et 4 mois de prise de produit.

### Critères de sécurité

Les critères de sécurité de cette étude, mesurés à la visite initiale et à 4 mois sont :
1) Le poids corporel (kg),
2) Les paramètres hémodynamiques : Fréquence Cardiaque (FC, bpm), Pression Artérielle Systolique (PAS, mmHg) et Pression Artérielle Diastolique (PAD, mmHg),
3) Les paramètres biologiques suivants :
   - Numération de la formule sanguine,
   - Fonction hépatique par le dosage des paramètres suivants : ASAT, ALAT et GGT,
   - Fonction rénale par le dosage de la créatinine.
4) Le nombre d'évènements indésirables par sujet apparus pendant toute la durée de l'étude ;
5) La proportion de sujets présentant au moins un évènement indésirable pendant toute la durée de l'étude.

### Critères exploratoires :

Les critères exploratoires de l'étude concernent les variations entre valeurs initiales et celles mesurées à 4 mois :
- Des paramètres obtenus par échographie pelvienne réalisée chez les patientes avec une endométriose profonde et / ou présence d'adénomyose,
- Du questionnaire EHP-30.

## Revendications

1. Combinaison d'une levure de l'espèce *Saccharomyces cerevisiae* de variété boulardii, de resvératrol et d'un extrait de curcuma comprenant au moins 15% en poids de curcuminoïdes, pour une utilisation dans le traitement ou la prévention des lésions d'endométriose chez un sujet souffrant ou susceptible de souffrir d'endométriose, par exemple pour ralentir le développement ou pour diminuer les lésions d'endométriose.

2. Combinaison d'une levure de l'espèce *Saccharomyces cerevisiae* de variété boulardii, de resvératrol et d'un extrait de curcuma comprenant au moins 15% en poids de curcuminoïdes, pour une utilisation selon la revendication 1, dans laquelle la combinaison est utilisée en outre pour le traitement ou la prévention d'un ou plusieurs symptômes ou troubles associés à l'endométriose choisis dans le groupe constitué par l'hypofertilité, l'infertilité, les douleurs pelviennes notamment les dysménorrhées et dyspareunies, les douleurs cataméniales notamment au niveau urinaire ou intestinale, les troubles urinaires et intestinaux, les métrorrhagies, les rectorragies, l'inflammation chronique péritonéale, l'hyperperméabilité intestinale, la fatigue chronique et les combinaisons de ceux.

3. Combinaison d'une levure de l'espèce *Saccharomyces cerevisiae* de variété boulardii, de resvératrol et d'un extrait de curcuma comprenant au moins 15% en poids de curcuminoïdes, pour une utilisation selon la revendication 1, dans laquelle la combinaison est utilisée pour normaliser la perméabilité intestinale, moduler le statut immunitaire, maintenir ou améliorer la fertilité et/ou diminuer le stress oxydatif chez le sujet souffrant ou susceptible de souffrir d'endométriose.

4. Combinaison d'une levure de l'espèce *Saccharomyces cerevisiae* de variété boulardii, de resvératrol et d'un extrait de curcuma comprenant au moins 15% en poids de curcuminoïdes, pour une utilisation selon l'une quelconque des revendications 1 à 3, dans laquelle la levure de l'espèce *Saccharomyces cerevisiae,* le resvératrol et l'extrait de curcuma, sont contenus dans une seule composition pharmaceutique, nutraceutique ou alimentaire ou sont répartis dans plusieurs compositions pharmaceutiques, nutraceutiques ou alimentaires séparées destinées à être administrées simultanément ou séquentiellement.

5. Combinaison d'une levure de l'espèce *Saccharomyces cerevisiae* de variété boulardii, de resvératrol et d'un extrait de curcuma comprenant au moins 15% en poids de curcuminoïdes, pour une utilisation selon l'une quelconque des revendications 1 à 4, dans laquelle le sujet souffrant d'endométriose a subi ou va subir un traitement chirurgical d'exérèse des lésions d'endométriose, un protocole médicamenteux de procréation médicalement assisté, notamment de fécondation *in vitro* et/ou est sous traitement contraceptif.

6. Combinaison d'une levure de l'espèce *Saccharomyces cerevisiae* de variété boulardii, de resvératrol et d'un extrait de curcuma comprenant au moins 15% en poids de curcuminoïdes, pour une utilisation selon l'une quelconque des revendications 1 à 5, dans laquelle la levure de l'espèce *Saccharomyces cerevisiae* de variété boulardii, le resvératrol et l'extrait de curcuma est inclus dans un complément alimentaire, éventuellement avec une ou plusieurs substances à but physiologique ou nutritionnel choisies de préférence parmi les vitamines, les oligoéléments, les minéraux, les prébiotiques, les probiotiques, les antioxydants, les acides aminés et leurs combinaisons, et de manière plus préférée parmi l'acide folique, l'acide 5-methyltetrahydrofolique, la vitamine B6, la vitamine B12, le zinc, le magnésium, le calcium, le sélénium, le manganèse, l'inuline, les fructo/galacto-oligosaccharides, la gomme d'acacia, la gomme guar, l'inositol et leurs combinaisons

7. Combinaison d'une levure de l'espèce *Saccharomyces cerevisiae* de variété boulardii, de resvératrol et d'un extrait de curcuma comprenant au moins 15% en poids de curcuminoïdes, pour une utilisation selon l'une quelconque des revendications 1 à 6, ladite combinaison étant administrée en association avec du zinc, de préférence sous forme de sel de bisglycinate, et avec de l'inuline.

8. Composition pharmaceutique, nutraceutique ou alimentaire, de préférence sous la forme d'un complément alimentaire, comprenant une combinaison d'une levure de l'espèce *Saccharomyces cerevisiae* de variété boulardii, de resvératrol et un extrait de curcuma, selon les rapports suivants :
- de 1 à 50% en poids de levure *Saccharomyces cerevisiae* de variété boulardii
- de 10 à 50 % en poids de resvératrol,
- de 1 à 50% en poids d'extrait de curcuma comprenant au moins 15% en poids de curcuminoïdes, pour 100% en poids de la somme totale de resvératrol, de curcumine et de *Saccharomyces cerevisiae* de variété boulardii ou pour 100% en poids total de la composition, ladite composition étant de préférence adaptée pour une utilisation dans la prise en charge des sujets souffrant ou susceptibles de souffrir d'endométriose.

9. Composition selon la revendication 8, comprenant :
- un ou plusieurs prébiotiques ; et/ou
- des vitamines, minéraux et/ou oligo-élements ; et/ou
- des poudres ou extraits de plantes, fruits, légumes, algues ou champignons ; et/ou un ou plusieurs probiotiques, de préférence ledit probiotique n'étant pas une bactérie du genre *Lactobacillus.*

10. Composition selon la revendication 8 ou 9, ladite composition se présentant sous la forme d'unités de dose, par exemple sous la forme de comprimés ou de gélules, chaque unité de dose comprenant :
- de 10 à 300 mg, de préférence de 25 à 250 mg de resvératrol,
- de 1 à 300 mg, de préférence de 5 à 25 mg d'un extrait de curcuma comprenant au moins 15% en poids de curcuminoïdes, et
- de 1×10⁷ CFU à 1×10¹¹ CFU, de préférence 1×10⁸ CFU à 1×10¹⁰ ou 1×10⁸ CFU à 1×10⁹ CFU levure *Saccharomyces cerevisiae* variété boulardii.

11. Composition selon la revendication 9 comprenant en outre de l'inuline et du zinc.

12. Composition selon la revendication 10 ou 11 **caractérisée en ce qu'**elle se présente sous la forme d'unités de dose comprenant :
- de 30 mg à 70 mg de resvératrol, de préférence trans-resvératrol,
- de 5 à 25 mg de curcuminoïdes sous la forme d'un extrait de curcuma associé à une cyclodextrine,
- de 1×10⁸ CFU à 1×10¹⁰, de préférence de 2×10⁸ CFU à 6×10⁸ CFU, par exemple de 3×10⁸ CFU à 5×10⁸ CFU de levure *Saccharomyces cerevisiae* de variété boulardii,
- en option de 10 mg à 100 mg, de préférence de 25 mg à 50 mg d'inuline, et
- en option de 1 mg à 15 mg de zinc (Zn2+) de préférence de 2 mg à 8 mg de zinc, par exemple introduit sous forme de bisglycinate de zinc.

13. Kit pharmaceutique, nutraceutique ou alimentaire comprenant l'association de deux compositions :
- une première composition comprenant une levure *Saccharomyces cerevisiae* de variété *boulardii* en une quantité de 1×10⁷ CFU à 1×10¹¹ CFU et
- une seconde composition comprenant du resvératrol en une quantité de 10 à 300 mg et un extrait de curcuma en une quantité de 1 à 300 mg, ledit extrait de curcuma comprenant au moins 15% en poids de curcuminoïdes,
ledit kit étant adapté pour une utilisation chez des sujets souffrant ou susceptibles de souffrir d'endométriose.

14. Combinaison selon l'une quelconque des revendications 1-7, composition selon l'une quelconque des revendications 8-12, ou kit selon la revendication 13 caractérisé(e) en ce que :
- l'extrait de resvératrol est apporté sous la forme d'un extrait végétal enrichi en trans-resvératrol, par exemple un extrait de renouée du Japon titré à au moins 95% en poids de trans-resvératrol et/ou
- l'extrait de curcuma comprend de la curcumine, de la déméthoxycurcumine et de la bisdéméthoxycurcumine, et/ou
- l'extrait de curcuma comprend au moins 55%, de préférence au moins 60% en poids de curcumine par rapport au poids total des curcuminoïdes présents dans l'extrait, et/ou
- l'extrait de curcuma est encapsulé dans une cyclodextrine, de préférence une gamma cyclodextrine, et/ou
- l'inuline est apportée sous la forme d'un extrait de racine de chicorée.

## Patentansprüche

1. Kombination einer Hefe der Spezies *Saccharomyces cerevisiae* der Variante boulardii, von Resveratrol und eines Kurkumaextrakts, der mindestens 15 Gew.-% Curcuminoide enthält, zur Verwendung bei der Behandlung oder Vorbeugung von Endometriose-Läsionen bei einer Person, die an Endometriose leidet oder anfällig dafür ist, an Endometriose zu leiden, beispielsweise um die Entwicklung zu verlangsamen oder Endometriose-Läsionen zu reduzieren.

2. Kombination einer Hefe der Spezies *Saccharomyces cerevisiae* der Variante boulardii, von Resveratrol und eines Kurkuma-Extrakts, der mindestens 15 Gew.-% Curcuminoide enthält, zur Verwendung nach Anspruch 1, wobei die Kombination zusätzlich zur Behandlung oder Vorbeugung eines oder mehrerer Symptome oder Störungen im Zusammenhang mit Endometriose verwendet wird, ausgewählt aus der Gruppe bestehend aus Subfertilität, Unfruchtbarkeit, Beckenschmerzen, insbesondere Dysmenorrhoe und Dyspareunie, Menstruationsschmerzen, insbesondere im Harn- oder Darmbereich, Harn- und Darmstörungen, Metrorrhagie, Rektum Blutungen, chronische Peritonealentzündung, intestinale Hyperpermeabilität, chronische Müdigkeit und Kombinationen davon.

3. Kombination einer Hefe der Spezies *Saccharomyces cerevisiae* der Variante boulardii, von Resveratrol und eines Kurkumaextrakts, der mindestens 15 Gew. % Curcuminoiden enthält, zur Verwendung nach Anspruch 1, wobei die Kombination verwendet wird zur Normalisierung der Darmpermeabilität , zur Modulierung des Immunstatus, der Aufrechterhaltung oder Verbesserung der Fruchtbarkeit und/oder Reduzierung des oxidativen Stresses bei einer Person, die an Endometriose leidet oder anfällig dafür ist, an Endometriose zu leiden.

4. Kombination einer Hefe der Spezies *Saccharomyces cerevisiae* der Variante boulardii, von Resveratrol und eines Kurkumaextrakts, der mindestens 15 Gew.-% Curcuminoide enthält, zur Verwendung nach einem der Ansprüche 1 bis 3, wobei die Hefe der Spezies *Saccharomyces cerevisiae,* das Resveratrol und der Kurkumaextrakt in einer einzigen pharmazeutischen, nutrazeutischen oder Lebensmittel-Zusammensetzung enthalten sind oder in mehreren separaten pharmazeutischen, nutrazeutischen oder Lebensmittelzusammensetzungen vertrieben werden, die zur gleichzeitigen oder aufeinanderfolgenden Verabreichung bestimmt sind.

5. Kombination einer Hefe der Spezies *Saccharomyces cerevisiae* der Variante boulardii, von Resveratrol und eines Kurkumaextrakts, der mindestens 15 Gew.-% Curcuminoide enthält, zur Verwendung nach einem der Ansprüche 1 bis 4, bei der die Person, die an Endometriose leidet, sich einer chirurgischen Behandlung zur Entfernung von Endometrioseläsionen unterzogen hat oder sich einer solchen unterziehen wird, einem medizinischen Protokoll zur medizinisch unterstützten Fortpflanzung, insbesondere In-vitro-Fertilisation, unterzogen hat oder sich einer solchen unterziehen wird und/oder sich in einer Verhütungsbehandlung befindet

6. Kombination einer Hefe der Spezies *Saccharomyces cerevisiae* der Variante boulardii, von Resveratrol und eines Kurkumaextrakts, der mindestens 15 Gew.-% Curcuminoide enthält, zur Verwendung nach einem der Ansprüche 1 bis 5, wobei die Hefe der Spezies *Saccharomyces cerevisiae* der Variante Boulardii, das Resveratrol und der Kurkuma-Extrakt, in einem Nahrungsergänzungsmittel enthalten sind, gegebenfalls mit einem oder mehreren Stoffen für physiologische oder Ernährungszwecke, vorzugsweise ausgewählt aus Vitaminen, Spurenelementen, Mineralien, Präbiotika, Probiotika, Antioxidantien, Aminosäuren und Kombinationen davon und bevorzugter aus Folsäure, 5-Methyltetrahydrofolsäure, Vitamin B6, Vitamin B12, Zink, Magnesium, Calcium, Selen, Mangan, Inulin, Fructo/Galacto-Oligosaccharide, Akaziengummi, Guarkernmehl, Inositol und Kombinationen davon.

7. Kombination einer Hefe der Spezies *Saccharomyces cerevisiae* der Variante boulardii, von Resveratrol und eines Kurkumaextrakts, der mindestens 15 Gew.-% Curcuminoide enthält, zur Verwendung nach einem der Ansprüche 1 bis 6, wobei diese Kombination in Kombination mit Zink, vorzugsweise in Form von Bisglycinatsalz, und mit Inulin verabreicht wird.

8. Pharmazeutische, nutrazeutische oder Lebensmittel-Zusammensetzung, vorzugsweise in Form eines Nahrungsergänzungsmittels, umfassend eine Kombination aus einer Hefe der Spezies *Saccharomyces cerevisiae* der Variante boulardii, Resveratrol und einem Kurkumaextrakt, gemäß den folgenden Verhältnissen:
- 1 bis 50 Gew.-% Hefe *Saccharomyces cerevisiae* der Variante Boulardii,
- 10 bis 50 Gew.-% Resveratrol,
- 1 bis 50 Gew.-% Kurkuma-Extrakt, der mindestens 15 Gew.-% Curcuminoide umfasst,
wobei die Gesamtsumme aus Resveratrol, Curcumin und Saccharomyces cerevisiae der Sorte Boulardii 100 Gew.-% ausmacht oder 100 Gew.-% der Gesamtsumme der Zusammensetzung, wobei die Zusammensetzung vorzugsweise zur Verwendung bei der Behandlung von Personen geeignet ist, die an Endometriose leiden oder die anfällig dafür sind, an Endometriose zu leiden.

9. Zusammensetzung nach Anspruch 8, umfassend:
- ein oder mehrere Präbiotika; und/oder
- Vitamine, Mineralien und/oder Spurenelemente; und/oder
- Pulver oder Extrakte aus Pflanzen, Früchten, Gemüse, Algen oder Pilzen; und/oder
ein oder mehrere Probiotika, wobei dieses Probiotikum vorzugsweise kein Bakterium der Gattung Lactobacillus ist.

10. Zusammensetzung nach Anspruch 8 oder 9, wobei diese Zusammensetzung in Form von Dosiseinheiten vorliegt, beispielsweise in Form von Tabletten oder Kapseln, wobei jede Dosiseinheit Folgendes umfasst:
- 10 bis 300 mg, vorzugsweise 25 bis 250 mg, Resveratrol,
- 1 bis 300 mg, vorzugsweise 5 bis 25 mg, eines Kurkumaextrakts, der mindestens 15 Gew.-% Curcuminoide enthält, und
- l×l0⁷ KBE bis 1×l0¹¹ KBE, vorzugsweise l×l0⁸ KBE bis l×l0¹⁰ oder l×l0⁸ KBE bis l×l0⁹ KBE Hefe *Saccharomyces cerevisiae* Variante boulardii.

11. Zusammensetzung nach Anspruch 9, weiterhin umfassend Inulin und Zink

12. Zusammensetzung nach Anspruch 10 oder 11, **dadurch gekennzeichnet, dass** sie in Form von Dosiseinheiten vorliegt, umfassend:
- 30 mg bis 70 mg Resveratrol, vorzugsweise trans-Resveratrol,
- 5 bis 25 mg Curcuminoide in Form eines Kurkumaextrakts assoziiert mit einem Cyclodextrin,
- l×l0⁸ KBE bis l×l0¹⁰, vorzugsweise 2×l0⁸ KBE bis 6×l0⁸ KBE, beispielsweise 3×l0⁸ KBE bis 5×l0⁸ KBE, der Hefe *Saccharomyces cerevisiae* der Variante Boulardii,
- wahlweise 10 mg bis 100 mg, vorzugsweise 25 mg bis 50 mg, Inulin, und
- wahlweise 1 mg bis 15 mg Zink (Zn2+), vorzugsweise 2 mg bis 8 mg Zink, beispielsweise in Form von Zinkbisglycinat.

13. Pharmazeutisches, nutrazeutisches oder Lebensmittel-Kit, umfassend die Kombination zweier Zusammensetzungen:
- eine erste Zusammensetzung, die eine Hefe *Saccharomyces cerevisiae* der Variante Boulardii in einer Menge von l×l0⁷ KBE bis l×l0¹¹ KBE umfasst, und
- eine zweite Zusammensetzung, die Resveratrol in einer Menge von 10 bis 300 mg und einen Kurkuma-Extrakt in einer Menge von 1 bis 300 mg umfasst, wobei dieser Kurkuma-Extrakt mindestens 15 Gew.-% Curcuminoide umfasst,
wobei dieses Kit zur Verwendung bei Personen geeignet ist, die an Endometriose leiden oder die anfällig dafür sind, an Endometriose zu leiden

14. Kombination nach einem der Ansprüche 1-7, Zusammensetzung nach einem der Ansprüche 8-12 oder Kit nach Anspruch 13, **dadurch gekennzeichnet, dass**:
- der Resveratrol-Extrakt in Form eines mit trans-Resveratrol angereicherten Pflanzenextrakts bereitgestellt wird, beispielsweise eines Extrakts aus japanischem Staudenknöterich, titriert auf mindestens 95 Gew.-% trans-Resveratrol und/oder
- der Kurkuma-Extrakt Curcumin, Demethoxycurcumin und Bisdemethoxycurcumin umfasst, und/oder
- der Kurkuma-Extrakt mindestens 55 Gew.-%, vorzugsweise mindestens 60 Gew.-% Curcumin enthält, bezogen auf das Gesamtgewicht der im Extrakt vorhandenen Curcuminoide, und/oder
- der Kurkuma-Extrakt in einem Cyclodextrin, vorzugsweise einem Gamma-Cyclodextrin, verkapselt ist und/oder
- das Inulin in Form von Zichorienwurzelextrakt bereitgestellt wird.

## Claims

1. Combination of a yeast from species *Saccharomyces cerevisiae* of the variety boulardii, resveratrol and a curcuma extract comprising at least 15% by weight of curcuminoids, for use in the treatment or prevention of endometriotic lesions in a subject suffering from endometriosis or susceptible to suffer from endometriosis, for example to slow down the development or to reduce the endometriotic lesions.

2. Combination of a yeast from species *Saccharomyces cerevisiae* of the variety boulardii, resveratrol and a curcuma extract comprising at least 15% by weight of curcuminoids, for use according to claim 1, in which the combination is further used in the treatment or prevention of one or more symptoms or disorders associated with endometriosis selected from the group consisting of hypofertility, infertility, pelvic pains in particular dysmenorrhea and dyspareunia, catamenial pains in particular at the urinary or intestinal level, urinary and intestinal disorders, metrorrhagia, proctorrhagia, chronic peritoneal inflammation, intestinal hyperpermeability, chronic fatigue and combinations thereof.

3. Combination of a yeast from species *Saccharomyces cerevisiae* of the variety boulardii, resveratrol and a curcuma extract comprising at least 15% by weight of curcuminoids, for use according to claim 1, in which the combination is used for normalizing intestinal permeability, modulating the immune state, maintaining or improving fertility and/or decreasing oxidative stress in a subject suffering from endometriosis or is susceptible to suffer from endometriosis.

4. Combination of a yeast from species *Saccharomyces cerevisiae* of the variety boulardii, resveratrol and a curcuma extract comprising at least 15% by weight of curcuminoids, for use according to any of claims 1 to 3, in which the yeast from *Saccharomyces cerevisiae* species, resveratrol and the curcuma extract, are contained in a single pharmaceutical, nutraceutical or food composition or are distributed in several separate pharmaceutical, nutraceutical or nutritional compositions intended to be administered simultaneously or sequentially.

5. Combination of a yeast from species *Saccharomyces cerevisiae* of the variety boulardii, resveratrol and a curcuma extract comprising at least 15% by weight of curcuminoids, for use according to any of claims 1 to 4, in which the subject suffering from endometriosis has undergone or is to undergo a surgical treatment of resection of the endometriotic lesions, a medicinal protocol of medically assisted reproduction, in particular of in vitro fertilization and/or is under contraceptive treatment.

6. Combination of a yeast from species *Saccharomyces cerevisiae* of the variety boulardii, resveratrol and a curcuma extract comprising at least 15% by weight of curcuminoids, for use according to any of claims 1 to 5, in which the yeast of the species *Saccharomyces cerevisiae* of the variety boulardii, resveratrol and the curcuma extract are included in a food supplement, optionally with one or more ingredients with a physiological or nutritional purpose preferably selected from vitamins, trace elements, minerals, prebiotics, probiotics, antioxidants, amino acids and combinations thereof, and more preferably from folic acid, 5-methyltetrahydrofolic acid, vitamin B6, vitamin B12, zinc, magnesium, calcium, selenium, manganese, inulin, the fructo/galacto-oligosaccharides, acacia gum, guar gum, inositol and combinations thereof.

7. Combination of a yeast from species *Saccharomyces cerevisiae* of the variety boulardii, resveratrol and a curcuma extract comprising at least 15% by weight of curcuminoids, for use according to any of claims 1 to 6, said combination being administered in combination with zinc, preferably in the form of the bisglycinate salt, and with inulin.

8. Pharmaceutical, nutraceutical or food composition, preferably in the form of a food supplement, comprising a combination of a yeast from *Saccharomyces cerevisiae* species of the variety boulardii, resveratrol and a curcuma extract, in the following proportions:
- from 1 to 50 % by weight of yeast *Saccharomyces cerevisiae* of the boulardii variety
- from 10 to 50 % by weight of resveratrol,
- from 1 to 50% by weight of curcuma extract comprising at least 15% by weight of curcuminoids,
for 100% by weight of the total sum of resveratrol, curcumin and *Saccharomyces cerevisiae* or for 100% of the total weight of the composition, said composition preferably being suitable for use in the management of subjects suffering from endometriosis or subjects susceptible to sufer from endometriosis.

9. Composition according to claim 8, comprising:
- one or more prebiotics; and/or
- vitamins, minerals and/or trace elements; and/or
- powders or extracts of plants, fruits, vegetables, algae or fungi; and/or
one or more probiotics, preferably said probiotic not being a bacterium of the genus *Lactobacillus.*

10. Composition according to claim 8 or 9, said composition being in the form of dosage units, for example in the form of tablets or capsules, each dosage unit comprising:
- from 10 to 300 mg, preferably from 25 to 250 mg of resveratrol,
- from 1 to 300 mg, preferably from 5 to 25 mg of a curcuma extract comprising at least 15% by weight of curcuminoids, and
- from 1×10⁷ CFU to 1×10¹¹ CFU, preferably 1×10⁸ CFU to 1×10¹⁰ or 1×10⁸ CFU to 1×10⁹ CFU *Saccharomyces cerevisiae* yeast of variety boulardii.

11. Composition according to claim 9 further comprising inulin and zinc.

12. Composition according to claim 10 or 11 **characterized in that** it is in the form of dosage units comprising:
- from 30 mg to 70 mg of resveratrol, preferably trans-resveratrol,
- from 5 to 25 mg of curcuminoids in the form of a curcuma extract associated with a cyclodextrin,
- from 1×10⁸ CFU to 1×10¹⁰, preferably from 2×10⁸ CFU to 6×10⁸ CFU, for example from 3×10⁸ CFU to 5×10⁸ CFU of *Saccharomyces cerevisiae* yeast of the boulardii variety,
- optionally from 10 mg to 100 mg, preferably from 25 mg to 50 mg of inulin, and
- optionally from 1 mg to 15 mg of zinc (Zn2+) preferably from 2 mg to 8 mg of zinc, for example introduced in the form of zinc bisglycinate.

13. Pharmaceutical, nutraceutical or nutritional kit comprising the combination of two compositions:
- a first composition comprising a yeast *Saccharomyces cerevisiae* preferably of the *boulardii* variety, preferably in an amount from 1×10⁷ CFU to 1×10¹¹ CFU and
- a second composition comprising resveratrol preferably in an amount from 10 to 300 mg and a curcuma extract in an amount from 1 to 300 mg, said curcuma extract comprising at least 15% by weight of curcuminoids,
said kit being suitable for use in subjects suffering from endometriosis or subjects susceptible to suffer from endometriosis.

14. Combination according to any one of claims 1-7, composition according to any one of claims 8-12, or kit according to claim 13 **characterized in that**:
- the resveratrol extract is supplied in the form of a plant extract enriched with trans-resveratrol, for example an extract of Japanese knotweed titrated to at least 95% by weight of trans-resveratrol and/or
- the curcuma extract comprises curcumin, demethoxycurcumin and bisdemethoxycurcumin, and/or
- the curcuma extract comprises at least 55% by weight, preferably at least 60% by weight of curcumin relative to the total weight of the curcuminoids present in the extract, and/or
- the curcuma extract is encapsulated in a cyclodextrin, preferably a gamma cyclodextrin, and/or
- inulin is supplied in the form of an extract of chicory root.
